# EUROPEAN PATENT APPLICATION

(11) **EP 2 946 792 A1**
(43) Date of publication of application: **25.11.2015**
(21) Application number: 13871530.5
(22) Date of filing: 24.07.2013
(51) Int. Cl.: A61K 45/00, A23L 1/30, A61K 31/58, A61K 45/06, A61P 25/00, A61P 25/28, A61P 43/00

(54) **THERAPEUTIC AGENT AND THERAPEUTIC METHOD RELATING TO 1,25D3-MARRS FOR NEUROLOGICAL DISEASE SUCH AS ALZHEIMER'S DISEASE& xA;**

(30) Priority: 21.01.2013 JP 2013008136
(71) Applicant: Resilio Company Limited, Minato-ku Tokyo 107-0052 (JP)
(72) Inventor: TOHDA, Chihiro, Imizu-shi Toyama 939-0351 (JP)
(74) Representative: Teipel, Stephan
(86) International application number: PCT/JP2013/070064
(87) International publication number: WO 2014/112145

(57) **Abstract**

A primary objective is to provide a drug for radical cure and a therapeutic method for diseases associated with axonal dysfunction (for example, Alzheimer's disease, dementia, Parkinson's disease, spinal cord injury, or brain contusion) based on the pathogenic mechanism thereof.

Provided is a compound having a stimulating activity on 1,25D₃-MARRS or a pharmaceutically acceptable salt thereof. Also provided is a technical idea relating to a pharmaceutical composition for disease treatment comprising a therapeutically effective amount thereof, a production method, a method for preventing or treating a disease, and a kit.

## Description

### Technical Field

The present invention relates to a compound having a stimulating activity on 1,25D₃-MARRS, which is one of the receptors for dihydroxy vitamin D3 (herein, may be abbreviated to DHVD3). More specifically, the present invention relates to a therapeutic drug or a therapeutic method which utilizes a stimulating activity on 1,25D₃-MARRS for diseases associated with axonal dysfunction (for example, Alzheimer's disease or the like). Herein, Alzheimer's disease may be abbreviated to AD.

The present invention also relates to a pharmaceutical composition and a food or drink for prevention and/or treatment of neurological diseases, which comprise a therapeutically effective amount of the compound having a stimulating activity on 1,25D₃-MARRS or a pharmaceutically acceptable salt thereof; and a method for producing the same. Further, the present invention also relates to a method for preventing or treating a neurological disease in a mammal including a human with use of the compound or a pharmaceutically acceptable salt thereof.

### Background Art

Alzheimer's disease (AD) is defined as progressive decline in cognitive function and as dysfunction which is not observed in normal aging process. Diagnostic information of AD is described in the Diagnostic and Statistical Manual of Mental Disorders, 4th Edition (DSM-IV) published by American Psychiatric Association.

Currently, the treatment of AD is limited to symptomatic therapy with use of symptom-improving drugs represented by acetylcholinesterase inhibitors, and drugs for radical cure which are capable of treating the disease itself and stopping the progression thereof have not yet been developed. For the creation of a drug for radical cure of AD, the clarification of the pathogenic mechanism and the development of a new method for controlling the etiologic factors are needed. Cholinergic hypothesis, Aβ hypothesis, tau hypothesis, etc. have been suggested regarding the pathogenic mechanism of AD, and numerous studies are conducted to identify the mechanism.

With the emerging role of acetylcholine in learning and memory, "cholinergic hypothesis of Alzheimer's disease" proposing that degeneration of cholinergic neurons in the basal forebrain and the associated loss of cholinergic neurotransmission in the brain cortex and other areas contribute significantly to the deterioration in cognitive function seen in patients with Alzheimer's disease has been argued (Non Patent Literature 1). Based on this action mechanism, acetylcholinesterase inhibitors, which inhibit degradation of acetylcholine in synapses in the brain, are marketed as a therapeutic drug for AD. Examples of the acetylcholinesterase inhibitors include donepezil, galanthamine, and rivastigmine.

Also, Aβ protein, which is a metabolite of amyloid precursor protein (hereinafter called APP), is considered to be greatly concerned with the degeneration and loss of neurons and the expression of cognitive deficits (Non Patent Literature 2 and 3). β secretase and γ secretase participate in the formation of Aβ proteins, and depending on the cleavage site of the protein, Aβ (1-38), which consists of 38 amino acids, Aβ (1-40) having two more amino acids at the C terminus, Aβ (1-42) having four more amino acids at the C terminus, etc. are produced. These Aβs are highly aggregative (Non Patent Literature 4) and are primary constituents of the senile plaques (Non Patent Literature 4, 5, 6, and 7). That is, such aggregates eventually change into insoluble precipitates and highly-concentrated neuritic plaques, which are the pathological features of AD (Non Patent Literature 8). Further, mutations in APP and presenilin genes observed in familial AD are known to increase these Aβ proteins (Non Patent Literature 9, 10, and 11). Therefore, compounds capable of lowering the Aβ production are expected to be promising drugs which retard progression of AD or prevent AD. Based on the expectation, creation of drugs, such as Aβ antibodies and secretase inhibitors, intended to lower the Aβ production has been attempted. Some candidate therapeutic drugs for AD based on the hypothesis are currently under clinical trials, and a certain level of efficacy in AD patients has been reported (Non Patent Literature 12 and 13).

As described above, the drugs currently used for AD patients in clinical practice can prevent or retard the onset or progression of AD, but cannot improve the cognitive function. That is, the current treatment of AD is limited to symptomatic therapy with use of symptom-improving drugs represented by acetylcholinesterase inhibitors, and drugs for radical cure which are capable of improving the disease itself have not yet been developed. For the creation of a drug for radical cure of AD, the development of a method for controlling the causative factors of neurological dysfunction is needed. In addition, providing a compound which suited to the new mechanism is truly desired.

### Citation List

### Non Patent Literature

[NPL 1] Francis, P. et al., J Neurol Neurosurg Psychiatry 66; 137-147, 1999
[NPL 2] Klein, WL. et al., Proceeding of the National Academy of Science USA, Sep. 2; 100 (18), 10417-10422, 2003
[NPL 3] Nitsch, RM. et al., Neuron, May 22, 38, 547-554, 2003 [NPL 4] Jarrett, JT. et al., Biochemistry, 32 (18), 4693-4697, 1993
[NPL 5] Glenner, GG. et al., Biochemical and Biophysical Research Communications, May 16, 120 (3), 885-890, 1984
[NPL 6] Masters, CL. et al., Proceeding of the National Academy of Science USA, Jun. 82 (12), 4245-4249, 1985
[NPL 7] Gong, Y. et al., PNAS 100; 10417-10422, 2003
[NPL 8] Hardy, J. et al., Science 297; 353-356, 2002
[NPL 9] Gouras, GK. et al., American Journal of Pathology, Jan. 156 (1), 15-20, 2000
[NPL 10] Scheuner, D. et al., Nature Medicine, Aug. 2 (8), 864-870, 1996
[NPL 11] Forman, MS. et al., Journal of Biological Chemistry, Dec. 19, 272 (51), 32247-32253, 1997
[NPL 12] Mount, C. et al., Nature Medicine 12; 780-784, 2006
[NPL 13] Siemers, ER. et al., Clinical Neuropharmacology, 30; 317-325, 2007
[NPL 14] Cho, S. et al., Experimental Neurology, 203; 274-278, 2007

### Summary of Invention

### Technical Problem

The objectives of the present invention are to provide a completely new mechanism which leads to the radical cure of AD, to provide, based on the mechanism, a compound which is suited to the mechanism, and to provide a method for the radical cure.

Other objectives of the present invention are to provide a compound which leads to the radical cure of not only AD but also other neurological diseases caused by axonal dysfunction, and to provide a method for the radical cure of the diseases.

### Solution to Problem

In light of the above circumstances, the present inventors thought that morphological and functional improvement of the dysfunctional axons in the brain would recover the memory ability, and searched for drugs having such an activity. Based on the idea that exploring the action mechanism of such drugs leads to new finding of "a molecule involved in the cure", which cannot be anticipated from the pathological analysis of AD, the inventors wholeheartedly conducted investigations.

As a result, the inventors obtained the surprising finding that diosgenin, which is a compound contained in some kinds of plants, such as herbal plants, is useful for the treatment of AD. The inventors conducted further investigations and reached the useful and unexpected finding that the effect of diosgenin in the treatment of AD is attributable to the stimulation and activation of 1, 25D₃-MARRS, which is one of the receptors for dihydroxy vitamin D3.

After repeated tests and investigations, the inventors found that diosgenin and other substances having a stimulating activity on 1,25D₃-MARRS are effective for not only AD but also other neurological diseases, such as spinal cord injury, dementia, Parkinson's disease, and brain contusion. The inventors conducted further investigations and completed the present invention.

That is, the present invention relates to the following.
[1] A compound for prevention and/or treatment of a neurological disease, the compound having a stimulating activity on 1,25D₃-MARRS; and/or a pharmaceutically acceptable salt thereof, with the exception of the case where the disease is Alzheimer's disease or spinal cord injury and the compound is denosomin.
[2] The compound according to the above [1] and/or a pharmaceutically acceptable salt thereof, wherein the compound having a stimulating activity on 1,25D₃-MARRS is diosgenin and/or a diosgenin derivative.
[3] The compound according to the above [1] and/or a pharmaceutically acceptable salt thereof, wherein the compound having a stimulating activity on 1,25D₃-MARRS is diosgenin and/or dioscin.
[4] The compound according to any of the above [1] to [3] and/or a pharmaceutically acceptable salt thereof, wherein the neurological disease is Alzheimer's disease, dementia, Parkinson's disease, spinal cord injury, or brain contusion.
[5] Use of the compound according to any of the above [1] to [3] and/or a pharmaceutically acceptable salt thereof for the production of a drug for preventing and/or treating a neurological disease.
[6] The use according to the above [5], wherein the neurological disease is Alzheimer's disease, dementia, Parkinson's disease, spinal cord injury, or brain contusion.
[7] The compound according to any of the above [1] to [3] and/or a pharmaceutically acceptable salt thereof for the use in the prevention and/or treatment of a neurological disease.
[8] The compound according to the above [7] and/or a pharmaceutically acceptable salt thereof, wherein the neurological disease is Alzheimer's disease, dementia, Parkinson's disease, spinal cord injury, or brain contusion.
[9] A pharmaceutical composition for the treatment of a neurological disease, the composition comprising a therapeutically effective amount of the compound according to any of the above [1] to [3] and/or a pharmaceutically acceptable salt thereof.
[10] The pharmaceutical composition according to the above [9], wherein the neurological disease is Alzheimer's disease, dementia, Parkinson's disease, spinal cord injury, or brain contusion.
[11] The pharmaceutical composition according to the above [9] or [10], the composition further comprising a therapeutically effective amount of one or more compounds known to be effective for the treatment or prevention of a disease or one or more pharmaceutically acceptable salts thereof.
[12] The pharmaceutical composition according to the above [11], wherein the one or more compounds known to be effective for the treatment or prevention of a disease or one or more pharmaceutically acceptable salts thereof are known to be effective for the treatment or prevention of a neurological disease.
[13] A method for preparing the pharmaceutical composition according to any of the above [9] to [12], the method comprising blending at least one carrier.
[14] A method for preventing and/or treating a neurological disease, the method comprising administering the compound according to any of the above [1] to [3] or a pharmaceutically acceptable salt thereof to a mammal including a human.
[15] The method according to the above [14], wherein the compound according to any of the above [1] to [3] or a pharmaceutically acceptable salt thereof is used in combination with one or more compounds known to be effective for the treatment or prevention of a neurological disease or one or more pharmaceutically acceptable salts thereof.
[16] The method according to the above [14] or [15], wherein the neurological disease is Alzheimer's disease, dementia, Parkinson's disease, spinal cord injury, or brain contusion.
[17] A kit used for preventing and/or treating a neurological disease, the kit comprising the compound according to any of the above [1] to [3] or a pharmaceutically acceptable salt thereof.
[18] The kit according to the above [17], comprising the compound according to any of the above [1] to [3] or a pharmaceutically acceptable salt thereof and a container.
[19] A method for activating 1, 25D₃-MARRS, the method comprising administering one or more kinds selected from the group consisting of diosgenin, a diosgenin derivative, denosomin, dihydroxy vitamin D3, and a pharmaceutically acceptable salt thereof.
[20] A method for preventing or treating Alzheimer's disease, the method comprising administering one or more kinds selected from the group consisting of diosgenin, a diosgenin derivative, dihydroxy vitamin D3, and a pharmaceutically acceptable salt thereof.
[21] A method for reducing amyloid plaques, tau deposition, tau precipitate, PHF-tau, or neurofibrillary tangles, the method comprising administering diosgenin, a derivative thereof, a pharmaceutically acceptable salt thereof, or an ester thereof to a mammal including a human.
[22] A method for suppressing Aβ (1-42) -induced axonal atrophy, the method comprising administering diosgenin, a derivative thereof, a pharmaceutically acceptable salt thereof, or an ester thereof to a mammal including a human.
[23] A method for activating a signaling pathway by the stimulation of 1,25D₃-MARRS, the method comprising administering diosgenin, a derivative thereof, a pharmaceutically acceptable salt thereof, or an ester thereof to a mammal including a human.
[24] A health food, a functional food, or a specified health food comprising the compound according to any of the above [1] to [3] and/or a pharmaceutically acceptable salt thereof.

### Advantageous Effects of Invention

According to the present invention, it is possible to provide a completely new mechanism which leads to the radical cure of AD, to provide, based on the mechanism, a compound and a pharmaceutical composition which is suited to the mechanism, and to provide a method for the radical cure.

Further, according to the present invention, it is possible to provide a compound and a pharmaceutical composition which lead to the radical cure of not only AD but also other neurological diseases caused by axonal dysfunction, and to provide a method for the radical cure.

### Brief Description of Drawings

Fig. 1 shows the effects of diosgenin on object recognition memory deficits and AD pathologies in 5XFAD mice (Alzheimer's disease model).
Fig. 2 shows the effects of diosgenin on axonal and presynaptic abnormalities associated with amyloid plaques.
Fig. 3 shows 1,25D₃-MARRS as a direct target protein of diosgenin.
Fig. 4 shows the docking simulation of diosgenin to 1,25D₃-MARRS.
Fig. 5 shows the effect of 1,25D₃-MARRS knockdown on diosgenin-induced axonal outgrowth.
Fig. 6 shows the effects of protein kinase inhibitors on diosgenin-induced axonal growth.
Fig. 7 shows the effect of the 1,25D₃-MARRS neutralizing antibody on diosgenin-induced axonal regeneration.
Fig. 8 shows the memory enhancing effect of diosgenin in normal mice.
Fig. 9 shows that the object recognition memory of normal mice was improved by intraperitoneally administered diosgenin.
Fig. 10 shows that the improvement of the object recognition memory of normal mice was not observed in a test where diosgenin was administered after the function of 1,25D₃-MARRS was inhibited by a neutralizing antibody.
Fig. 11 shows that denosomin is also an exogenous stimulator of 1,25D₃-MARRS.
Fig. 12 shows the enhancing effect of orally administered dioscin on the object recognition memory of normal mice.
Fig. 13 shows the enhancing effect of orally administered dioscin on the object recognition memory of 5XFAD mice.
Fig. 14 shows the enhancing effect of diosgenin on the hindlimb motor function of spinal cord injury (SCI) mice.

### Description of Embodiments

Hereinafter, the invention will be described in more detail.

An embodiment of the present invention relates to a compound for prevention and/or treatment of a neurological disease, the compound having a stimulating activity on 1,25D₃-MARRS; and/or a pharmaceutically acceptable salt thereof.

The 1,25D₃-MARRS (1,25D₃-membrane-associated, rapid response steroid-binding protein) is one of the receptors for dihydroxy vitamin D3, and is also referred to as Pdia3 (protein disulfide isomerase family A, member 3), ERp57 (endoplasmic reticulum stress protein 57), GRP58 (glucose-regulated protein 58), etc. Hereinafter, dihydroxy vitamin D3 may be stated as DHVD3.

In the present invention, the neurological disease is not particularly limited as long as axonal dysfunction is involved in the disease. Included are preferably neurological diseases in which 1, 25D₃-MARRS is responsible for the treatment, and more preferably neurological diseases in which stimulating and activating 1,25D₃-MARRS leads to a certain observable beneficial action.

The neurological disease may be a traumatic neurological disease or a neurodegenerative disease, and is not particularly limited. Examples thereof include spinal cord injury, brain contusion, Alzheimer's disease, Parkinson's disease, and dementia. In the present invention, the dementia does not include Alzheimer's disease, and preferably include cerebrovascular dementia, Levy body dementia, frontotemporal dementia, and Pick's disease.

The source of the technical idea of the present invention is the findings that stimulating and activating 1,25D₃-MARRS induces extension of axons, such as axons in the brain, and that such extension enables the prevention and/or treatment of a neurological disease, such as Alzheimer's disease.

The compound of the present invention having a stimulating activity on 1,25D₃-MARRS includes pharmaceutically acceptable salts thereof, optical isomers thereof and mixtures of the optical isomers, solvates thereof, and crystal polymorphs thereof. The compound having a stimulating activity on 1,25D₃-MARRS is not particularly limited, and the examples thereof include diosgenin, dioscin, DHVD3, denosomin, etc. Among them, especially preferred are diosgenin and/or dioscin because of their easy availability and other advantages.

In the present invention, the "pharmaceutically acceptable salt" is not particularly limited as long as it is a pharmaceutically acceptable salt of the compound having a stimulating activity on 1,25D₃-MARRS. Specific examples of the salt include hydrogen halides (for example, hydrofluoride, hydrochloride, hydrobromide, hydroiodide, etc.), inorganic salts (for example, sulfate, nitrate, perchlorate, phosphate, carbonate, bicarbonate, etc.), organic carboxylates (for example, acetate, oxalate, maleate, tartrate, fumarate, citrate, etc.), organic sulfonates (for example, methanesulfonate, trifluoromethanesulfonate, ethanesulfonate, benzenesulfonate, toluenesulfonate, camphorsulfonate, etc.), amino acid salts (for example, aspartate, glutamate, etc.), quaternary amine salts, alkali metal salts (for example, sodium salt, potassium salt, etc.), alkaline earth metal salts (for example, magnesium salt, calcium salt, etc.), and the like.

The compound having a stimulating activity on 1,25D₃-MARRS to be used may be a commercially available product or alternatively be produced by a publicly known method or an equivalent method.

The compound having a stimulating activity on 1,25D₃-MARRS can be found by screening which uses detection of luminescence or fluorescence resulting from binding of the compound to 1,25D₃-MARRS, for example. Specifically, the screening may be performed as follows.

### Detection of luminescence resulting from 1,25D₃-MARRS binding

Primary-cultured cerebral cortical neurons (SD, E17) are seeded at a density of 0.5 × 10⁵ cells/well onto a 96-well white plate. Three days later, the cells are treated with DHVD3 alone (control) or with a combination of DHVD3 and a compound to be tested (compound X) at 37°C for 10 minutes. The cells are fixed in 4% paraformaldehyde and for the detection of only DHVD3 bound to the surfaces of the cells, incubated with a monoclonal antibody against DHVD3 (1:2000, an Acris Antibodies, Herford, Germany) in Triton-X-free PBS at 4°C for 20 hours. Using a goat anti-mouse IgG antibody (1:2000) labeled with Horseradish peroxidase as a second antibody, the chemiluminescence of the antigen-antibody complex is detected using a multi-plate reader. From the value of the luminescence intensity of the control, the corresponding value of the compound X is deducted to determine the intensity of the luminescence resulting from the 1,25D₃-MARRS binding by the tested compound.

### Detection of fluorescence resulting from 1,25D₃-MARRS binding

Primary-cultured cerebral cortical neurons (SD, E17) are seeded at a density of (0.5 to 0.8) × 10⁵ cells/well onto a 96-well black plate. Three days later, the cells are treated with biotin-labeled DHVD3 alone (control) or with a combination of biotin-labeled DHVD3 and a compound to be tested (compound X) at 37°C for 10 minutes. The cells are washed, treated with Alexa Fluor 488-labeled streptavidin, left at room temperature for 1 hour, and washed again. The fluorescence of the biotin-streptavidin complex is detected using a multi-plate reader. From the value of the fluorescence intensity of the control, the corresponding value of the compound X is deducted to determine the intensity of the fluorescence of the 1,25D₃-MARRS binding by the tested compound.

Said diosgenin is a steroid sapogenin and present in some kinds of plants, such as herbal plants, including *Dioscorea* rhizome, *Trigonella* spp., *Polygonatum* spp., *Smilax* spp., etc. Diosgenin has been reported to have various effects, such as anticancer effect (Yan, L.L. etal., Exp Oncol, 31, 27-32, 2009), anti-food allergy effect (Huang, C.H. et al., Planta Med, 75, 1300-1305, 2009), suppressing effect on oxidative stress-induced memory deficits caused by galactose administration (Chiu, C.S. et al., Am J Chin Med, 39, 551-563, 2011) and anti-diabetic neuropathy (Kang, T.H. et al., Biol Pharm Bull, 34, 1493-1498, 2011). In addition, diosgenin is known for skin whitening effect (JP-2010-535758 W), skin improvement effect including wrinkle removal (JP-2009-501209 W and JP-2007-016013 A), hair growth effect (JP-2006-273754 A), etc.

For example, as the diosgenin, a commercially available product or an extract from a natural product may be used, as will be described later.

Herein, a diosgenin derivative is a diosgenin equivalent which is a compound having a stimulating activity on 1,25D₃-MARRS. The diosgenin derivative to be used may be a commercially available product, a product obtained by a publicly known method or an equivalent method, or an extract from a natural product. For example, the diosgenin derivative may be a diosgenin equivalent obtainable by a chemical modification of diosgenin, for example, substituent introduction or substituent conversion, or a diosgenin glycoside extracted from a natural product.

Said dioscin is a saponin contained in yam etc. and is a diosgenin glycoside. Dioscin can be preferably used as a diosgenin derivative compound having a stimulating activity on 1,25D₃-MARRS.

Said denosomin is a newly synthesized compound represented by the following formula (I): 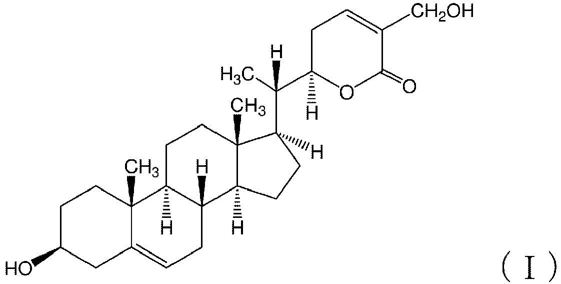 and is reported to improve spinal cord injury (Teshigawara, K. et al., Br J Pharmacol. 2013, Feb; 168(4), 903-919). Also, in Matsuya, Y. et al., Org Lett. 2009 Sep 3; 11(17), 3970-3973, it is reported that denosomin is a novel anti-Alzheimer's disease medicine candidate. However, regarding the signaling mechanism of denosomin, nothing has been known so far.

Said DHVD3 represented by the following formula (II): 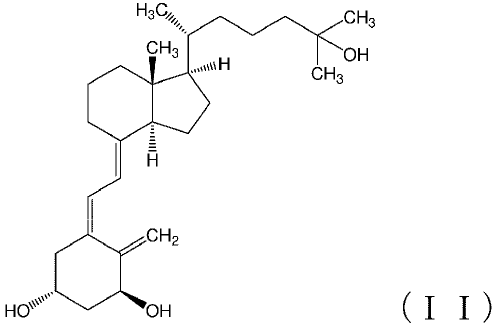 is the hormonally active form of vitamin D and is also referred to as calcitriol.

In the above embodiment of the present invention, the case where the disease is spinal cord injury or Alzheimer's disease and the compound is denosomin may be excluded.

Another embodiment of the present invention relates to a pharmaceutical composition for treating neurological diseases, the composition comprising a therapeutically effective amount of a compound having a stimulating activity on 1,25D₃-MARRS and/or a pharmaceutically acceptable salt thereof.

The neurological disease and the compound having a stimulating activity on 1,25D₃-MARRS and/or a pharmaceutically acceptable salt thereof may be the same as above.

The pharmaceutical composition of the present invention can be formulated by a conventional method, but the formulation method is not limited thereto.

Preferred examples of the dosage form of the pharmaceutical composition include a tablet, a powder, a fine granule, a granule, a dry syrup, a coated tablet, an orally-disintegrating tablet, a chewable tablet, a capsule, a soft capsule, a syrup, an oral solution, a troche, a jelly, a inhalation, a suppository, an injection, an ointment, an eye drop, an eye ointment, a nasal drop, an ear drop, a cataplasm, a lotion, a liquid for external use, a spray, an aerosol for external use, a cream, a gel, a tape, a buccal tablet, a sublingual tablet, a vaginal suppository, a vaginal tablet, a rectal soft capsule, etc. In the formulation, conventionally used agents, such as an excipient, a binder, a disintegrant, a coating agent, a lubricant, a colorant, and a corrigent; and as needed, a stabilizer, an emulsifier, an absorption promoter, asurfactant, a pH adjuster, a preservative, an antioxidant, etc. may be used. The formulation can be performed in the usual manner using components usually used as raw materials of a pharmaceutical composition. The present invention further includes a method for preparing a pharmaceutical composition, the method comprising using at least one carrier.

The components (carriers) used for the formulation are not particularly limited, and examples thereof include plant or animal oils, for example, soybean oil, beef tallow, and synthetic glyceride; hydrocarbons, for example, liquid paraffin, squalane, and solid paraffin; ester oils, for example, octyldodecyl myristate and isopropyl myristate; higher alcohols, for example, cetostearyl alcohol and behenyl alcohol; silicon resin; silicone oil; surfactants, for example, polyoxyethylene fatty acid ester, sorbitan fatty acid ester, glycerin fatty acid ester, polyoxyethylene sorbitan fatty acid ester, polyoxyethylene hydrogenated castor oil, and polyoxyethylene-polyoxypropylene block copolymer; water-soluble polymers, for example, hydroxyethyl cellulose, polyacrylic acid, a carboxyvinyl polymer, polyethylene glycol, polyvinyl pyrrolidone, and methyl cellulose; lower alcohols, for example, ethanol and isopropanol; polyhydric alcohols, for example, glycerin, propylene glycol, dipropylene glycol, and sorbitol; sugars, for example, glucose and sucrose; inorganic powders, for example, silicic anhydride, magnesium aluminum silicate, and aluminum silicate; and purified water. As the excipient, for example, lactose, cornstarch, saccharose, d-glucose, mannitol, sorbitol, crystalline cellulose, silicon dioxide, etc. may be used. As the binder, for example, polyvinyl alcohol, gelatin, methyl cellulose, ethyl cellulose, gum arabic, tragacanth, gelatin, shellac, hydroxypropyl methylcellulose, hydroxypropylcellulose, polyvinyl pyrrolidone, polyvinyl acetal diethylamino acetate, corn starch, etc. may be used. As the disintegrant, for example, corn starch, low substituted hydroxypropylcellulose, crospovidone, crystalline cellulose, precipitated calcium carbonate, croscarmellose sodium, calcium citrate, dextrin, pectin, carboxymethylcellulose calcium, etc. may be used. As the lubricant, for example, magnesium stearate, talc, polyethylene glycol, light anhydrous silicic acid, sucrose fatty acid ester, etc. may be used. As the colorant, those allowed to be added to pharmaceutical products may be used. As the corrigent, cocoa powder, menthol, aromatic powder, mentha oil, borneol, a cinnamon powder, etc. may be used.

For example, to prepare an oral formulation, the compound or a salt or ester thereof, or a hydrate thereof as an active ingredient, together with an excipient, and with, for example, a binder, a disintegrant, a lubricant, a colorant, a corrigent, etc. as needed, is formulated into, for example, a powder, a fine granule, a granule, a tablet, a coated tablet, a capsule, etc. in the usual manner. In the case of a tablet or a granule, needless to say, it is of no matter that the tablet or the granule is coated with sugar or another appropriate ingredient as needed. In the case of a syrup, a formulation for injection, etc., the active ingredient, together with, for example, a pH adjuster, a solubilizing agent, a isotonic agent, etc. and with a solubilizing agent, a stabilizing agent, etc. as needed, is formulated in the usual manner. In the case of an external formulation, the preparation method is not particularly limited, and the formulation can be prepared in the usual manner. As the base material, various raw materials usually used for drugs, quasi drugs, cosmetics, etc. can be used. Examples of the raw material include plant or animal oils, mineral oils, ester oils, waxes, higher alcohols, fatty acids, silicone oils, surfactants, phospholipids, alcohols, polyhydric alcohols, water soluble polymers, acrylic adhesives, clay minerals, purified water, etc. As needed, a pH adjuster, an antioxidant, a chelating agent, an antiseptic antifungal agent, a colorant, a flavor, etc. can also be used. Further, as needed, a component having a differentiation-inducing activity, for example, a blood circulation enhancer, a germicide, an anti-inflammatory agent, a cell activator, vitamins, an amino acid, a moisturizer, a keratolytic agent, etc. can also be blended.

The administration route of the pharmaceutical composition of the present invention is not particularly limited, and may be oral or parenteral. Examples of the parenteral administration include, for example, rectal administration, nasal administration, intrapulmonary administration, injection administration (for example, intravenous administration, intraspinal administration, epidural administration, intramuscular administration, subcutaneous administration, intraperitoneal administration, intraarterial administration, intraarticular administration, intracardiac administration, intracapsular administration, intracutaneous administration, intralesional administration, intraocular administration, intrapleural administration, subarachnoid administration, intrauterine administration, intraventricular administration), etc.

In a particularly preferred embodiment of the present invention, when the compound having a stimulating activity on 1,25D₃-MARRS is diosgenin, it is intraspinally or intravenously administered, and when the compound is dioscin, it is orally administered.

The amount of the compound having a stimulating activity on 1,25D₃-MARRS or a pharmaceutically acceptable salt thereof contained in the pharmaceutical composition is not particularly limited, but is preferably a sufficient amount for the treatment, improvement, or resolution of the symptoms associated with neurological diseases.

The dosage amount of the pharmaceutical composition of the present invention varies with the degree of the symptoms, the age, the sex, the body weight, the route of administration, the type of the salt, the type of disease, and the like. Typically, in the case of an adult, the compound having a stimulating activity on 1,25D₃-MARRS or a pharmaceutically acceptable salt thereof is orally administered in an amount of about 2 mg to 10 g, preferably about 5 mg to 5 g, and more preferably about 10 mg to 1 g per day, or administered by injection in an amount of about 2 mg to 10 g, preferably about 5 mg to 5 g, and more preferably about 10 mg to 1 g per day. The daily dosage may be given as a single dose or divided into several doses.

In an embodiment of the present invention, the pharmaceutical composition may be used in combination with one or more compounds known to be effective for the treatment or prevention of a disease or one or more pharmaceutically acceptable salts thereof. The disease is not particularly limited, but is preferably a neurological disease, and more preferably AD.

In an embodiment of the present invention, when the neurological disease is AD, the compound having a stimulating activity on 1,25D₃-MARRS or a pharmaceutically acceptable salt thereof may be used in combination with one or more compounds known to be effective for the treatment or prevention of AD or the symptoms thereof.

Examples of the compound known to be effective for the treatment or prevention of AD or the symptoms thereof include compounds having the following action mechanisms for the treatment of diseases caused by amyloid β (Aβ), for example, AD, senile dementia, Down's syndrome, amyloidosis, etc.

Specific examples thereof include compounds having the action mechanism of choline esterase inhibitors (for example, donepezil, huperzine A, tacrine, rivastigmine, and galantamine); AMPA receptor antagonists (for example, 1,2-dihydropyridine compounds, such as 3-(2-cyanophenyl)-5-(2-pyridyl)-1-phenyl-1,2-dihydropyridin e-2-one); NMDA receptor antagonists (for example, memantine); acetylcholine release stimulants (for example, pramiracetam and aniracetam); calcium channel agonists (for example, nefiracetam) ; free radical scavengers (for example, EGb 761) ; platelet activator antagonists (for example, EGb 761) ; platelet aggregation antagonists (for example, EGb 761 and triflusal) ; insulin sensitizers (for example, rosiglitazone); peroxisome proliferator-activated receptor agonists (for example, rosiglitazone); peroxisome proliferator-activated receptor γ agonists (for example, rosiglitazone); monoamine oxidase B inhibitors (for example, rasagiline, selegiline, and procaine); carnitine acetyltransferase stimulants (for example, levacecarnine); NSAIDs (for example, cyclooxygenase-2-inhibitors, such as triflusal and celecoxib); nerve growth factor agonists (for example, xaliproden and FPF 1070); β amyloid inhibitors (for example, tarenflurbil, tramiprosate, and leuprorelin-D); immunomoderators (for example, tarenflurbil, immune globulin, and icosapentethyl ester); NF-κB inhibitors (for example, tarenflurbil); thyrotropin-releasing hormones (for example, taltirelin); dopamine D2 receptor antagonists (for example, risperidone); serotonin 2 receptor antagonists (for example, risperidone); muscarinic M1 receptor agonists (for example, cevimeline); α1-adrenergic receptor agonists (for example, modafinil); serotonin 3 receptor antagonists (for example, alosetron); dopamine D2 receptor agonists (for example, aripiprazole); dopamine D2 receptor antagonists (for example, aripiprazole); serotonin 1A receptor agonists (for example, aripiprazole); serotonin 2A receptor antagonists (for example, aripiprazole); glucocorticoid antagonists (for example, mifepristone); progesterone antagonists (for example, mifepristone); HMG-CoA reductase inhibitors (for example, atorvastatin and simvastatin); adenosine uptake inhibitors (for example, propentofylline); phosphodiesterase inhibitors (for example, propentofylline); acetylcholine receptor agonists (for example, choline alfoscerate); membrane-permeation enhancers (for example, choline alfoscerate); cannabinoid 1 receptor antagonists (for example, rimonabant); cannabinoid receptor agonists (for example, dronabinol); angiogenesis inhibitors (for example, paclitaxel); immunosuppressants (for example, paclitaxel); tubulin antagonists (for example, paclitaxel); thromboxane A synthase inhibitors (for example, triflusal); antioxidants (for example, idebenone); α adrenergic receptor antagonists (for example, nicergoline) ; estrogen antagonists (for example, conjugated estrogens and trilostane); 3-β hydroxysteroid dehydrogenase inhibitors (for example, trilostane); signaling pathway inhibitors (for example, trilostane); melatonin receptor agonists (for example, ramelteon); immunostimulants (for example, immune globulin, icosapentethyl ester, and procaine) ; HIV entry inhibitors (for example, procaine) ; sodium channel antagonists (for example, procaine); microtubule inhibitors (for example, CPH 82); glycine NMDA agonists (for example, cycloserine); adenosine A1 receptor antagonists (for example, KW 3902); ATPase stimulants (for example, triacetyluridine); mitochondrial functional enhancers (for example, triacetyluridine); growth hormone-releasing factor agonists (for example, tesamorelin); butyl choline esterase inhibitors (for example, bisnorcymserine); α adrenergic receptor antagonists (for example, nicergoline); NO synthase type II inhibitors (for example, arundic acid); chelating agents (for example, PBT 2); amyloid fibril formation inhibitors (for example, TTP488, PF 4494700); seretonin 4 receptor agonists (for example, PRX 03140); seretonin 6 receptor antagonists (for example, SB 742457); benzodiazepine receptor inverse agonists (for example, radequinil) ; Ca channel antagonists (for example, safinamide); nicotine receptor agonists (for example, ispronicline); BACE inhibitors (for example, CTS 21166); or the like.

Further, specific examples of the compounds include donepezil, huperzine A, tacrine, rivastigmine, galantamine, pramiracetam, aniracetam, nefiracetam, EGb761, rosiglitazone, rasagiline, levacecarnine, celecoxib, 3-(2-cyanophenyl)-5-(2-pyridyl)-1-phenyl-1,2-dihydropyridin e-2-one, talampanel, becampanel, memantine, xaliproden, tarenflurbil, tramiprosate, leuprorelin-D, taltirelin, risperidone, cevimeline, modafinil, alosetron, aripiprazole, mifepristone, atorvastatin, propentofylline, choline alfoscerate, FPF 1070 (CAS No. 143637-01-8), rimonabant, dronabinol, docosahexaenoic acid, paclitaxel, triflusal, idebenone, nicergoline, conjugated estrogens, trilostane, simvastatin, selegiline, ramelteon, immune globulin, icosapentethyl ester, procaine, CPH 82, cycloserine, KW 3902 (CAS No. 136199-02-5), triacetyluridine, estrogen dementia therapeutics (e. g., MIGENIX, Vancouver, Canada), tesamorelin, bisnorcymserine, nicergoline, arundic acid, PBT 2, TTP488, PF 4494700, PRX 03140, SB 742457, radequinil, safinamide, ispronicline, CTS 21166, bapineuzumab, NP 031112, (2S,3aS,7aS)-1{[(R,R)-2-phenylcyclopropyl]carbonyl}-2-[(thi azolidine-3-yl)carbonyl]octahydro-1H-indole, citalopram, venlafaxine, levprorelin, prasterone, peptide T (CAS No. 53-43-0), besipiridine, lexipafant, stacofylline, SGS 742 (CAS No. 123690-78-8), T 588 (CAS No. 142935-03-3), nerispiridine, dexanabinol, sabcomeline, GTS 21 (CAS No. 156223-05-1), CX516 (CAS No. 154235-83-3), ABT 089 (CAS No. 161417-03-4), anapsos, tesofensine, SIB 1553A (i.e., 4-[[2-(1-methyl-yl-2-pyrrolidinyl)ethyl]thia]phenol), ladostigil, radequinil, GPI 1485, ispronicline, arundic acid, MEM 1003 (i.e., 3-isopropyl-5-(2-methoxyl)4-(2-chloro-3-cyanophenyl)-2,6-di methylpyridine-3,5-dicarboxylase), V 3381 (i.e., 2-(2,3-dihydro-1H-indene-3-ylamino)acetamide hydrochloride), farampator, paliroden, prasterone-paladin, urocortin, DP b99 (i.e., 2,2'-(ethylenedioxy)bis(2,1-phenylene)bis[N-[2-[2-(octyloxy )ethoxy]-2-oxoethyl]imino]bis(acetic acid)), capserod, DU 125530, bapineuzumab, AL 108 (i.e., L-Asparaginyl-L-alanyl-L-prolyl-L-valyl-L-seryl-L-isoleucyl -L-prolyl-L-glutamine), DAS 431, DEBIO 9902, DAR 100, mitoquinone, IPL 455903 (i.e., 5(S)-[3-(cyclopenthyloxy)-4-methoxyphenyl]-3(S)-(3-methylbe nzyl)piperidine-2-one), E2CDS, PYM 50028, PBT 2, lecozotan, SB 742457, CX 717, AVE 1625 (i.e., 1-(bis(4-chlorophenyl)methyl)-3-((3,5-difluorophenyl)(methy lsulfonyl)methylene)azetidine), LY 450139 (i.e., N2-[2(s)-hydroxy-3-methylbutyryl]-N1-[3-methyl-2-oxo-2,3,4, 5-tetrahydro-1H-3-benzazepine-1(S)-yl]-L-alaninamide), EM 1421 (i.e., 4,4'-[(2R,3S)-2,3-dimethylbutane-1,4-diyl]bis(1,2-dimethoxy benzene), SRN 001, TTP 488, PRX 03140, dimebolin, glycine-proline-glutamate, C105, AL 208, MEM 3454, AC 1202, L 830982, LY 451395 (i.e., (R)-N-[2-[4'-(methylsulfonamidemethyl)biphenyl-4-yl]propyl] propane-2-sulfonamide), MK 0249, LY 2062430, diethylnorspermine, neboglamine, S 18986, SA 4503 (CAS No. 165377-44-6), GRI 1, S 17092 (i.e., (2S,3aS,7aS)-1{[(R,R)-2-phenylcyclopropyl]carbonyl}-2-[(thi azolidine-3-yl)carbonyl]octahydro-1H-indole), SL 251188, EUK 189, R 1450, 6,6-dimethyl-3-(2-hydroxyethyl)thio-1-(thiazole-2-yl)-6,7-d ihydro-2-benzothiophene-4(5H)-one, CERE 110, dexefaroxan, CAD 106, HF 0220, HF 0420, EHT 0202, VP 025,
MEM 1414, BGC 201259 (i.e., N,N-dimethylcarbamic acid, 4-[1(S)-(methylamino)-3-(4-nitrophenoxy)propyl]phenyl ester), EN 100, ABT 834, ABT 239 (i.e., 4-[2-[2-[(2R)-2-methylpyrrolidinyl]ethyl]-benzofuran-5-yl]b enzonitrile), SGS 518, R 1500, C 9138, SSR 180711, alfatradiol, R 1577, T 817MA (i.e., 1-[3-[2-(1-benzothiophene-5-yl)ethoxy]propyl]azetidine-3-ol maleate), CNP 1061 (i.e., 4-methyl-5-(2-nitrooxyethyl)thiazole), KTX 0101 (i.e., β-hydroxybutyric acid sodium salt), GSK 189254 (i.e., 6-[3-cyclobutyl-2,3,4,5-tetrahydro-1H-benzo[d]azepin-7-ylox y]-N-methylnicotinamide), AZD 1080, ACC 001, PRX 07034, midazolam, R-phenserine, AZD 103 (CAS No. 488-59-5), SN 522, NGX 267 (CAS No. 503431-81-0), N-PEP-12, RN 1219, FGLL, AVE 8112, EVT 101, NP 031112, MK 0752, MK 0952, LX 6171, PAZ 417, AV965, PF 3084014, SYN 114, GSI 953, SAM 315, SAM 531, D-serine, leteprinim potassium, BR16A (CAS No. 149175-77-9), RPR 107393 (CAS No. 190841-57-7), NXD 2858, REN 1654, CDD 0102, NC 1900 (CAS No. 132925-74-7), ciclosporin, NCX 2216 (i.e., (E)-4-(nitrooxy)butyl-3-[4-[2-(2-fluorobiphenyl-4-yl)propan oyloxy]-3-methoxypheny]acrylate), NXD 3109, NXD 1191, ZSET 845 (i.e., 3,3-diphenylimidazo[1,2-a]pyridine-2-(3H)-one), ET 002, NT 13, RO 638695 (i.e., [1,6-(1,6-dioxohexyl)]dipyrrolidine-(2R)-carboxylic acid), bisnorcymserine, BA 1016, XD 4241, EUK 207 (i.e., (SP-5-13)-(acetato-κO)[13,16,19,22-tetraoxa-3,6-diaza-tricy clo[21.3.18,12]octacosa-1(27),2,6,8,10,12(28),23,25-octaene -27,28-diolate(2-)-κN3, κN6, κO27, κO28]magnesium salt), LG 617 inhibitors, ZSET 1446, PAN 811, F 14413 (i.e., 2-[5-fluoro-2(S)-methoxy-2,3-dihydro-1,4-benzodioxine-2-yl] -4,5-dihydro-1H-imidazole), FP 7832 (i.e., N-[2-(5-methoxy-1-nitroso-1H-indole-3-yl)ethyl]acetamide), ARA 014418 (i.e., N-(4-methoxybenzyl)-N'-(5-nitro-1,3-thiazole-2-yl)urea), AZD 3102, KP 544 (i.e., 2-amino-5-(4-chlorophenylethynyl)-4-(4-trans-hydroxy-cycloh exylamino)pyrimidine), DP 155, 5-chloro-N-[3-[2-(dimethylamino)ethyl]-1H-indole-5-yl]napht halene-2-sulfonamide, TAK 070,
huperzine, N-[2-(3,5-dimethyladamant-1-yl)ethyl]acetamide hydrochloride, 6-[4-[(dimethylamino)methyl]-5-ethyl-2-methoxypheny]pyridin -2-amine, 4,6-diphenyl-3-(4-(pyrimidine-2-yl)piperazine-1-yl)pyridazi ne, N-[(1S,2R)-3-(3,5-difluorophenyl)-1-hydroxy-1-[(5S,6R)-5-me thyl-6-(neopentyloxy)molpholine-3-yl]propane-2-yl]acetamide hydrochloride, N-[(1R,2S)-3-(3,5-difluorophenyl)-1-hydroxy-1-[(2R,4R)-4-ph enoxypyrrolidine-2-yl]propane-2-yl]-3-[(R)-2-(methoxymethyl )pyrrolidine-1-carbonyl]-5-methylbenzamide, R 1589, midafotel, phenserine, coluracetam, physostigmine, cipralisant, nitroflurbiprofen, PPI 1019 (i.e., 3α, 5β, 7α, 12α)-trihydroxycholan-24-oyl-L-leucyl-L-valyl-L-phenylalan yl-L-phenylalanyl-L-alanine), dapsone, MDL 100453 (CAS No. 129938-34-7), NS 377, midaxifylline, propofol phosphate, metrifonate, ceronapril, tenilsetam, sufoxazine, seglitide, ebiratide, nebracetam, milacemide, iododoxorubicin, SM 10888 (CAS No. 129297-21-8), U 80816 (CAS No. 138554-11-7), YM 954 (CAS No. 132041-85-1), SUT 8701 (CAS No. 123577-73-1), apovincamine, FR 121196 (CAS No. 133920-65-7), LY 274614 (CAS No. 136109-04-1), CL 275838 (CAS No. 115931-65-2), igmesine, K 7259 (CAS No. 133667-88-6), vinconate, itasetron, CL 287663 (CAS No. 125109-98-0), WAY 100289 (CAS No. 136013-69-9), SR 46559A (CAS No. 137733-33-6), GYKI 46903 (CAS No. 142999-59-5), L 670548 (CAS No. 121564-89-4), Y 29794 (CAS No. 129184-48-1), AF 125 (CAS No. 7631-86-9), KFM 19 (CAS No. 133058-72-7), ST 796 (i.e., (S)-3-[3-(trifluoromethyl)benzoyl)amino]hexahydroazepin-2-o ne, RU 33965 (CAS No. 122321-05-5), SDZ 210086 (i.e., (-)-1',2(S)-dimethylspiro[1,3-dioxane-4,4'-piperidine]), L 689660 (CAS No. 144860-79-7), L 689560 (CAS No. 139051-78-8), ST 618 (i.e., 1-(6,7-dimethoxy-1,2,3,4-tetrahydro-2-naphthyl)-4-hydroxy pyrrolidine-2-one), U 74500A (CAS No. 110101-65-0), GEA 857 (CAS No. 120493-42-7), BIBN 99 (CAS No. 145301-48-0), DX 9366, ONO 1603 (CAS No. 114668-76-7), MDL 102234 (CAS No. 137766-81-5), P 9939 (CAS No. 157971-37-4), PD 140532 (CAS No. 157971-39-6), azetirelin, MR16728 (CAS No. 147614-21-9), dabelotine, MDL 102503 (i.e., 8-[1(R)-methyl-2-phenylethyl]-1,3-dipropyl-7H-xanthine), PD 141606 (i.e., (±)-(Z)-3-(3-phenyl-2-propynyloxyimino)-1-azabicyclo[2.2.1] heptane), SNK 882 (CAS No. 152221-12-0), L 696986 (CAS No. 141553-45-9), tazomeline, LY 235959 (CAS No. 137433-06-8), 2-(2-thioxopyrrolizine-1-yl)acetamide, AK 30 NGF, ABT 418 (CAS No.147402-53-7), itameline, HUP 13, sibopirdine, KST 5452 (CAS No. 157998-88-4), TJ 54, U92798 (i.e., 7-[4-[bis(4-fluorophenyl)methyl]perhydro-1,4-diazepine-1-yl methyl]-4-isopropyl-2-methoxy-2,4,6-cycloheptatriene-1-one), U 92032(CAS No. 142223-92-5),
3-(sulfamoyloxy)estra-1,3,5(10)-triene-17-one, P 11012 (CAS No. 164723-36-8), A 82695 (CAS No. 147388-86-1), FR 76659 (CAS No. 116904-25-7), apaxifylline, CX 417, 7 MEOTA (CAS No. 5778-80-3), BU 4514N (CAS No. 151013-39-7), pregnenolone, mexidol, ST 857 (CAS No. 154755-63-2), RU 49041 (CAS No. 123828-80-8), RU 35929 (CAS No. 111711-47-8), P 878184, P 128 (CAS No. 157716-52-4), eurystatin A, eurystatin B, LK 12, NBI 108, NBI 107, NBI 117, L 705106, bacoside A + B, clausenamide, SM 21 (CAS No. 155156-22-2), alaptide, RS 17017 (i.e., 1-(4-amino-5-chloro-2-methoxypheny)-5-(1-piperidinyl)-1-pen tanone hydrochloride), AF 150 (S) (i.e., (S)-[1-methyl-piperidine-4-spiro-(2'-methyl thiazoline)]), RO 153505 (CAS No. 78771-13-8), PV 113 (i.e., 1,2,3,4-tetrahydropyrrole-[1,2-a]-pyrazine), arisugacin, A 98284 (i.e., 2(R)-(3-methylxazole-5-yl)quinuclidine), AP 5 (CAS No. 136941-85-0), BD 1054, SDZ NDD 094 (i.e., bis-(2 -(2-methylimidazole-1-yl]methyl)-pyridine-tris(hydrogen-fum arate), AZ 36041 (CAS No. 173324-76-0), quilostigmine, A 84543 (i.e., 3-[1-methylpyrrolidine-2-(S)-ylmethoxy]pyridine fumarate), BTG 4247 (i.e., (2-[2-chloroethoxy[4-(dimethylamino)phenyl]phosphoryl]-acet ohydrazine), CGP 50068 (CAS No. 158647-49-5), cerebrocrast, desferri-nordanoxamine, isolichenan, MHP 133 (i.e., 3-(N,N-dimethoxycarbamoyloxy)-1-methyl-2-(4-phenyl-semicarb azomethyl)pyridium chloride), FR 152558 (CAS No. 151098-08-7), GVS 111 (CAS No. 157115-85-0), P11149 (CAS No. 164724-79-2), PDC 008004, KST 2818 (CAS No. 158623-26-8), KST 5410 (CAS No. 158623-27-9), RU 52583 (CAS No. 123829-33-4), PD 151832 (CAS No. 149929-39-5), UCL 1199 (i.e., 4-[2-[(5-nitropyridine-2-ylsulfanil)ethyl]-1H-imidazole), isovanihuperzine A, SIB 1765F (CAS No. 179120-52-6), JWS USC 751X (i.e., 3-[[[2-[[(5-dimethylaminoethyl)-2-furanyl]methyl]thio]ethy] amino]-4-nitropyridazine), GR 175737 (i.e., 3-(4-chlorobenzyl)-5-[2-(1H-imidazole-4-yl)ethyl]-1, 2, 4-oxadiazole), KS 505A (CAS No. 131774-53-3), ZTTA 1 (i.e., N-benzyloxycarbonyl-thiopropyl-thiopropynal-dimethylacetal), AGN 190837 (CAS No. 136527-40-7), P 10358 (188240-59-7), WAY 131256 (CAS No. 174001-71-9), DBO 83 (i.e., 3-(6-chloropyrazine-3-yl)-diazabicyclo[3.2.1]octane dihydrochloride monohydrate), FUB 181 (CAS No. 152029-80-6), RJR 2557, WSU 2088, LVV-haemorphin-7, M 40 (i.e., galanin[1-12]-Pro3-(Ala-Leu)2-Ala-NH2), SIB 1757, SKF 74652 (i.e., [5-chloro-2-(4-methoxypheny)-3-benzofuranyl][4-[3-(dimethyl amino)-propoxy]phenyl]methanone), CGP 71982, SCH 57790 (i.e., 4-cyclohexyl-α-[4-[[4-methoxypheny]sulfinyl]phenyl]-1-pipe razineacetonitrile), Putrescine-D-YiAbeta11, DU 14 (i.e., p-O-(sulfamoyl)-N-tetradecanoyltyramine), CLZ 4, SL 340026, PPRT 424, ciproxifan, UR 1827 (i.e., 2-(1-benzylpiperidine-4-yl)-1-[4-(5-methylpyrimidine-4-ylam ino)phenyl]-1-ethanone), caproctamine, TGS 20 (i.e., L-pyroglutamil-D-alanine amide), PG 9 (i.e., α-tropanyl 2-[(4-bromo)phenyl]propionate),
TEI 3356 (i.e., (16S)-15-deoxy-16-hydroxy-16-methyl-9-(O)-methano-DELTA6(9 α)-prostaglandin I1), LY 392098 (i.e., thiophene, 3-[(2-methylethy-2)sulfonylaminopropyl-2]phenyl-4-yl-), PG 1000, DM 232, NEPP 11 (i.e., 12-iso-15-deoxy-18-(4-methyl)phenyl-13,14-dihydro-delta 7-prostaglandin A1 methylester), VA 100 (i.e., (2,3-dihydro-2-[[(4-fluorobenzoyl)amino]ethyl]-1-methyl-5-p henyl-1H-1,4-benzodiazepine), VA 101 (i.e., t(2,3-dihydro-2-[[(2-thienylcarbonyl)amino]ethyl]-1-methyl-5-phenyl-1H-1,4-benzodiazepine)), NC 111585 (i.e., (3S)-1,3-bis-[3-[(3-azabicyclo[2.2.2]octanyl)-1,2,5-thiadia zole-4-yloxyl-1-propyne-1-yl]benzene, 2L-(+)-tartate), IN 201, imoproxifan, kanokodiol, picroside I, picroside II, DM 235 (i.e., 1-(4-benzoylpiperazine-1-yl)propane-1-one), monoclonal antibody 10D5, JLK2, JLK6, JLK7, DAPT (i.e., N-[N-(3,5-difluorophenacetyl)-L-alanyl]-S-phenylglycine t-butylester), huperine X, SGS 111 (i.e., (S)-ethyl 2-[1-(2-phenylacetyl)pyrrolidine-2-carboxamide]acetate), NP 7557, C 9136, C 7617, R 1485, rofecoxib, velnacrine, montirelin, lazabemide, ORG 2766 (CAS No. 50913-82-1), sabeluzole, adafenoxate, CAS No. 9061-61-4, ipidacrine, bemesetron, idazoxan, linopirdine, selfotel, suritozole, milameline, xanomeline, TJ960, fasoracetam, eptastigmine, ensaculin, zanapezil, posatirelin, zacopride, RS 86 (CAS No. 3576-73-6), ORG 5667 (CAS No. 37552-33-3), RX 77368 (CAS No. 76820-40-1), BMS 181168 (CAS No. 123259-91-6), BY 1949 (CAS No. 90158-59-1), AWD 5239 (CAS No. 109002-93-9), YM 796 (171252-79-2), aloracetam, CI 933 (CAS No. 91829-95-7), ST 793 (CAS No. 99306-37-3), cebaracetam, zifrosilone, talsaclidine, alvameline, JTP 2942 (148152-77-6), OPC 14117 (CAS No. 103233-65-4), elziverine, AP 521 (i.e., N-(1,3-benzodioxol-5-ylmethyl)-1,2,3,4-tetrahydro[1]benzoth ieno[2,3-c]pyridine-3(R)-carboxamide hydrochloride), S 8510 (CAS No. 151466-23-8), JTP 4819 (CAS No. 162203-65-8), icopezil, SC 110, FK 960 (CAS No. 133920-70-4), DMP 543 (CAS No. 160588-45-4), ganstigmine, CI 1017 (i.e., (R)-(-)-(2)-1-azabicyclo[2.2.1]heptane-3-one, O-(3-(3'-methoxypheny)-2-propionyl)-oxime maleate), T 82 (i.e., 2-[2-(1-benzylpiperidine-4-yl)ethyl]-2,3-dihydro-9-methoxy-1H-pyrrolo[3,4-b]quinolin-1-one hemifumarate), NGD 971, vaccine of aspartyl-alanyl-glutamyl-phenylalanyl-arginyl-histidyl-aspa rtyl-seryl-glycyltyrosyl-glutamyl-valyl-histidyl-histidyl-glutaminyl-lysyl-leucyl-valyl-phenylalanyl-phenylalanyl-ala nyl-glutamyl-aspartyl-valyl-glycyl-seryl-asparaginyl-lysyl-glycyl-alanyl-isoleucyl-isoleucyl-glycylleucyl-methionyl-va lyl-glycyl-glycyl-valyl-valyl-isoleucyl-alanine, PBT 1 (CAS No. 130-26-7), TCH 346, FK 962 (i.e., N-(1-acetylpiperidine-4-yl)-4-fluorobenzamide), voxergolide, KW 6055 (CAS No. 63233-46-5), thiopilocarpine, ZK 93426 (CAS No. 89592-45-0), SDZ NVI 085 (CAS No. 104195-17-7), CI 1002 (CAS No. 149028-28-4),
Z 321 (CAS No. 130849-58-0), mirisetron, CHF 2060 (i.e., N-heptylcarbamic acid 2,4a,9-trimethyl-2,3,4,4a,9,9a-hexahydro-1,2-oxazino[6,5-b] indole-6-yl ester-L-tartarate), gedocarnil, terbequinil, HOE 065 (CAS No. 123060-44-6), SL 650102, GR 253035, ALE 26015, SB 271046 (i.e., 5-chloro-N-(4-methoxy-3-piperazine-1-yl-phenyl)-3-methyl-2-benzothiophene sulfonamide), iAbeta5, SCH 211803 (i.e., 3-chlorophenyl[4-[1-[1-(2-amino-3-methylbenzoyl)-4-piperidi nyl]-4-piperidinylmethyl]phenyl]sulfone), EVT 301, α-linolenic acid/linoleic acid, Kamikihi-to, siagoside, FG 7142 (CAS No. 78538-74-6), RU 47067 (CAS No. 111711-92-3), RU 35963 (CAS No. 139886-03-6), FG 7080 (CAS No. 100332-18-1), E 2030 (CAS No. 142007-70-3), transforming growth factor β-1,A 72055 (i.e., 2',1-dimethylspiro[piperidine-4,5'-oxazolidine]-3'-carboxya ldehyde), NS626, dimiracetam, GT 3001, GT 2501, GT 2342, GT 2016 (CAS No. 152241-24-2), ORG 20091 (CAS No. 141545-50-8), BCE 001 (CAS No. 95678-81-2), CGP 35348 (CAS No. 123690-79-9), WAY 100635 (CAS No. 146714-97-8), E 4804 (CAS No. 162559-34-4), LIGA 20 (CAS No. 126586-85-4), NG 121 (i.e., 2-4,8-dimethyl-3(E),7(E)-nonadienyl]-3,5-dihydroxy-2-methyl -3,4,7,9-tetrahydro-2H-fluoro[3,4-h]-1-benzopyran-7-one), MF 247 (i.e., N-[10-(diethylamino)decyl]carbamic acid (3aS,8aR)-1,3a,8-trimethyl-1,2,3,3a,8,8a-hexahydropyrrolo[2 ,3-b]indole-5-yl ester), JTP 3399 (i.e., N-benzyl-2(S)-[2(S)-(phenoxyacetyl)pyrrolidine-1-yl-carbony l]pyrrolidine-1-carboxamide), KF 17329, thioperamide, F 3796 (i.e., 1-[2-(1-benzylpiperidine-4-yl)ethyl]-3-[3,4-(methylene-diox y)benzoyl]thiourea), GT 4001, GT 4002, FPL 14995 (CAS No. 123319-03-9), RU 34332 (CAS No. 137157-58-5), SR 96777A (CAS No. 115767-94-7), SIB T1980, NS 649 (CAS No. 146828-02-6), PD 142505 (CAS No. 149929-08-8), GYKI 52466 (CAS No. 102771-26-6), RO 246173 (CAS No. 159723-57-6), SCH 50911 (CAS No. 160415-07-6), Z 4105 (CAS No. 119737-52-9), RS 67333 (CAS No. 168986-60-5), NS 1546, ZM 241385 (CAS No. 139180-30-6), RO 249975 (i.e., [1S,3S(2'S),5R]-3-(1-benzyl-5-oxopyrrolidine-2-ylmethyl)-5-(1H-imidazole-5-ylmethyl)cyclohexane-1-acetamide), AF 185 (i.e., 8-methyl-3-(2-propynyl)-1,3,8-triazaspiro [4,5]decane-2,4-dione), CEP 427, CX 423, CX 438, CX 480, CDP-ethanolamine, GT 4003, GT 4011, GT 5011, MS 430 (CAS No. 122113-44-4), MBF 379 (i.e., [3,3-bis(hydroxymethyl)-8-hydroxy-3,4-dihydro-2H-1,4-benzox azine-5-yl][3',5'-dihydroxy-4'-(2-oxo-2-phenylethoxy)phenyl ]methanone), NGD 187 (CAS No. 163565-48-8), DUP 856, MR 3066, MF 8615 (i.e., 5-amino-6-chloro-4-hydroxy-3,4-dihydro-1H-thiopyrano-[3,4-b ]quinoline), himbacine, ABS 300, RJR 2403 (CAS No. 538-79-4), MF 268 (CAS No. 174721-00-7), RO 465934 (i.e., N,N-dimethylcarbamic acid 3-(2-cyclohexyl)-2,3,3a,4,5,9b-hexahydro-1H-benzo[e]indole-6-yl ester), NS 393, RGH 2716 (CAS No. 134069-68-4),
WIN 678702 (12,12-bis(3-furyl)-6,11-dihydro-6,11-ethanobenzo[b]quinoli zinium chloride), RS 66252 (i.e., 1-butyl-2-[(2'-(2H-tetrazole-5-yl)-biphenyl-4-yl)methyl]-1H -indole-3-carboxylic acid), AIT 034 (CAS No. 138117-48-3), NG 012 (CAS No. 131774-53-3), PD 142012 (CAS No. 5778-84-7), GT 4054, GT 4077, GT 4035, P 26 (CAS No. 152191-74-7), RGH 5279 (i.e., (-)-(13aR,13bS)-13a-ethyl-2,3,5,6,13a,13b-hexahydro-1H-indo lo[3,2,1-de]pyrido[3,2,1-ij][1,5]naphthyridine-12-carboxyli c acid 2-acetoxyethyl ester), AIT 083, CeNeS, estradiol (i.e., 1,3,5(10)-estratriene-3,17β-diol), WAY 132983 ((3R,4R)-3-(3-hexasulfanylpyrazine-2-yloxy)-1-azabicyclo[2. 2.1]heptane hydrochloride), ABS 205, ABS 401, SX 3507 (i.e., 3-(3-propyl-1,2,4-oxadiazole-5-yl)quinoxaline 2(1H)-one), ARR 17779 (i.e., (-)-spiro[1-azabicyclo[2.2.2]octaene-3,5-oxazolidine]-2-one ), XE 991 i.e., 10,10-bis(4-pyridylmethyl)anthracene10(9H)-one), phenethylnorcymserine, RO 657199, RJR 1781 (i.e., R(+)-2-(3-pyridyl)-1-azabicyclo[2.2.2.]octane), RJR 1782 (i.e., S(-)-2-(3-pyridyl)-1-azabicyclo[2.2.2.]octane), gilatide, tolserine, TC 2559 (i.e., (E)-N-methyl-4-[3-(5-ethoxypyridine)yl]-3-butene-1-amine), ER 127528 (i.e., 1-(3-fluorobenzyl)-4-[(2-fluoro-5,6-dimethoxy-1-indanone-2-yl)methyl]piperidine hydrochloride), thiatolserine, targacept, axonyx, cymserine, thiacymserine, monoclonal antibody 266, Apan-CH, DP 103, SPI 339 (i.e., 4-[3-(4-oxo-4,5,6,7-tetrahydroindole-1-yl)propionylamino]be nzoic acid ethyl ester), S 37245 (i.e., 4-(1,4-benzodioxane-5-yl)-1-[3(S)-hydroxy-5-nitro-indane-2-yl]-piperazine), LLG 88, AZD 2858, trometamol, AN 240, NG 002 (i.e., 5-hydroxy-5-(2-hydroxy-1-methylethyl)-4-methoxyfuran-2(5H)-one), UCB 29427 (i.e., 2-cyclopropyl-4-(cyclobutylpropylamino)-6-(morpholino)-1,3, 5-triazine), TRH-SR, RO 401641 (CAS No.122199-02-4), MPV 1743AIII (CAS No. 150586-64-4), IDRA 21 (CAS No. 22503-72-6), CEP431, ACPD (CAS No. 67684-64-4), CT 3577 (i.e., 3,7-dimethyl-1-[11-(3,4,5-trimethoxybenzylamino)-11-oxounde cyl]xanthine), CT 2583, NXD 9062, Desferri-nordanoxamine, DP b99, PBT 1, T 817MA, Alfatradiol (CAS No. 57-91-0), AL 108, SL 650102, RS 67333 (CAS No. 168986-60-5), RS 17017, SGS 518, SYN 114, SB 271046, RO 657199, PRX 07034, Suritozole (CAS No. 110623-33-19), Terbequinil (CAS No. 113079-82-6), FG 7142 (CAS No. 78538-74-6), RU 34332 (CAS No. 137157-58-5), SX 3507, RO 153505 (CAS No. 78771-13-8), RU 33965 (CAS No. 122321-05-5), S 8510 (CAS No. 151466-23-8), Sabeluzole (CAS No. 104383-17-7), Cerebrocrast (CAS No. 118790-71-9), NS 626, NS 649 (CAS No.146828-02-6), U92032 (CAS No. 142223-92-5), MEM 1003, U 92798, RGH 2716 (CAS No. 134069-68-4), Safinamide (CAS No. 133865-89-1), AZD 0328, MEM 63908, ABT 418 (CAS No. 147402-53-7), ARR 17779, RJR 2403 (CAS No. 538-79-4), TC 2559, A 82695 (CAS No. 147388-86-1), A 84543, A 98284, DBO 83, RJR 2557, SIB 1765F (CAS No. 179120-52-6), GTS 21 (CAS No. 156223-05-1), MEM 3454, SIB 1553A, EVP 6124, SSR 180711,
ABT 089 (CAS No. 161417-03-4), ABT 107, ABT560, TC 5619, TAK 070, N-[(1S,2R)-3-(3,5-difluorophenyl)-1-hydroxy-1-[(5S,6R)-5-me thyl-6-(neopentyloxy)morpholine-3-yl]propane-2-yl]acetamide hydrochloride, 6-fluoro-5-(2-fluoro-5-methylphenyl)-3,4-dihydropyridine, 2-amino-6-[2-(3'-methoxybiphenyl-3-yl)ethyl]-3,6-dimethyl-5 ,6-hydroxypyrimidine-4(3H)-one, AZD 1080, ARA014418, XD 4241, Z 321 (CAS No. 130849-58-0), ONO 1603 (CAS No. 114668-76-7), JTP 3399, Eurystatin A (CAS No. 137563-63-4), Eurystatin B (CAS No. 137563-64-5), P 128 (CAS No. 157716-52-4), Y 29794 (CAS No. 129184-48-1), ZTTA 1, JTP 4819 (CAS No. 162203-65-8), monoclonal antibody 266, duloxetine, escitalopram oxalate, fluoxetine, fluvoxamine maleate, paroxetine, sertraline, dapoxetine, desvenlafaxine, sibutramine, nefazodone, milnacipran, desipramine, duloxetine, and bicifadine.

The composition of the present invention may be provided, if desired, in the form of a kit which comprises a container, such as a pack or a dispenser, capable of containing one or more unit dosage forms containing an active ingredient.

In the present invention, different pharmaceutical compositions may also be combined in the form of a kit. The kit may comprise two or more kinds of different pharmaceutical compositions. For example, the kit comprises the compound of the present invention and one or more compounds known to be useful for the treatment or prevention of AD, and/or the compound of the present invention and a compound which exerts medicinal benefits in the treatment of a disease other than AD. The kit usually comprises a container, such as a divided bottle or a divided foil packet, for separately containing the different compositions, but the different compositions may also be contained in a single, undivided container. The form of a kit is particularly advantageous when different components are preferably administered in different dosage forms (e.g., oral and parenteral), when different components are administered at different dosage intervals, or when titration of the individual components of the combination is desired by the prescribing physician.

The pack may be a blister pack, for example, which may comprise a metal foil or a plastics foil, for example. Blister packs are well known in the packaging industry and are widely used for the packaging of pharmaceutical unit dosage forms (tablets, capsules, and the like). In general, a blister pack preferably consists of a sheet of a relatively stiff material covered with a foil of a transparent plastic material. During the packaging process, recesses are formed in the plastic foil. The recesses have a size and a shape suitable for each tablet or capsule to be packed. Next, the tablets or capsules are placed in the recesses, and the plastic foil is sealed at the face opposite from the direction in which the recesses were formed with use of a sheet of relatively stiff material. As a result, the tablets or capsules are sealed in the cavities between the plastic foil and the sheet. Preferably, the strength of the sheet is such that, when a pressure is manually applied to a cavity, the sheet can be broken at the site of the cavity to form an opening which allows the tablet or capsule to be removed from the blister pack. The tablet or capsule can be removed via the opening.

A package insert for administration, a product insert, etc. can be attached to the pack or the dispenser. The pack, the dispenser and other containers used can be adapted to the notifications issued by the government agency or the authorities which regulate medicinal production, use, or sale.

An embodiment of the present invention relates to a food or drink, a feed, a food additive, a feed additive, or the like, which comprises a compound having a stimulating activity on 1,25D₃-MARRS and/or a pharmaceutically acceptable salt thereof.

Hereinafter, the food or drink of the present invention will be described.

To the food or drink of the present invention, one or more kinds of food additives generally used in foods or drinks may be added, and examples thereof include a sweetener, a colorant, a preservative, a thickener, an antioxidant, a color improver, a decolorant, an antifungal agent, a gum base, a bittering agent, an enzyme, a brightener, an acidulant, a seasoning, an emulsifier, a fortifier, an agent for production, a flavor, a spice extract, etc. The food or drink of the present invention includes a health food, a functional food, a food for specified health use, and a food for babies, toddlers, pregnant or nursing mothers, the elderly, or the sick.

The form of the food or drink of the present invention is not particularly limited. Specific examples thereof include so-called dietary supplements, such as a tablet, a granule, a powder, and a health drink. Other examples include drinks, such as tea drink, refreshing drink, soda, nutritional drink, fruit juice, and lactic acid drink; noodles, such as buckwheat noodle, wheat noodle, Chinese noodle, and instant noodle; sweets and bakery products, such as candy, candy, gum, chocolate, snack, biscuit, jelly, jam, cream, baked goods, and bread; fishery or livestock products, such as fish sausage, ham, and sausage; dairy, such as processed milk and fermented milk; fats, oils, and processed foods thereof, such as salad oil, oil for frying, margarine, mayonnaise, shortening, whipped cream, and dressing; seasonings, such as sauce and dipping sauce; retort pouch foods, such as curry, stew, rice-bowl cuisine, porridge, and rice soup; and frozen desserts, such as ice cream, sherbet, and shaved ice.

The intake of the food or drink of the present invention is not particularly limited and may be appropriately set depending on the form of the food or drink; the age, the sex, the state, etc. of the ingester as a subject; the kind of the functional substance supported by or mixed with the carrier of the present invention; and/or other conditions.

Another embodiment of the present invention relates to use of the compound and/or a pharmaceutically acceptable salt thereof for the production of a drug for preventing and/or treating a neurological disease, the compound having a stimulating activity on 1,25D₃-MARRS. The neurological disease, the compound having a stimulating activity on 1,25D₃-MARRS and/or a pharmaceutically acceptable salt thereof, the dosage form of the drug, the administration route, the content of the compound having a stimulating activity on 1,25D₃-MARRS and/or a pharmaceutically acceptable salt thereof as an active ingredient in the drug, and the dosage may be the same as above.

Another embodiment of the present invention relates to a method for reducing amyloid plaques, tau deposition, tau precipitate, PHF-tau, or neurofibrillary tangles, the method comprising administering a compound having a stimulating activity on 1,25D₃-MARRS and/or a pharmaceutically acceptable salt thereof or an ester thereof etc. to a mammal. In this embodiment, the compound having a stimulating activity on 1,25D₃-MARRS is preferably one or more kinds selected from the group consisting of diosgenin, dioscin, and denosomin. In this embodiment, the mammal as the subject of administration is not particularly limited, and the examples include a human, an ape, a hamadryas baboon, a chimpanzee, a mouse, a rat, a guinea pig, a hamster, a rabbit, a cat, a dog, a sheep, a goat, a pig, and a cow. The dosage form of the drug, the administration route, the content of the compound having a stimulating activity on 1,25D₃-MARRS and/or a pharmaceutically acceptable salt thereof as an active ingredient in the drug, and the dosage may be the same as above.

Another embodiment of the present invention relates to a method for suppressing Aβ (1-42)-induced axonal atrophy, the method comprising administering a compound having a stimulating activity on 1,25D₃-MARRS and/or a pharmaceutically acceptable salt thereof or an ester thereof, etc. to a mammal. In this embodiment, the compound having a stimulating activity on 1,25D₃-MARRS is preferably one or more kinds selected from the group consisting of diosgenin, dioscin, and denosomin. In this embodiment, the mammal as the subject of administration is not particularly limited, and the examples include a human, an ape, a hamadryas baboon, a chimpanzee, a mouse, a rat, a guinea pig, a hamster, a rabbit, a cat, a dog, a sheep, a goat, a pig, and a cow. The dosage form of the drug, the administration route, the content of the compound having a stimulating activity on 1,25D₃-MARRS and/or a pharmaceutically acceptable salt thereof as an active ingredient in the drug, and the dosage may be the same as above.

Another embodiment of the present invention relates to a method for activating a signaling pathway by the stimulation of 1, 25D₃-MARRS, the method comprising administering a compound having a stimulating activity on 1,25D₃-MARRS and/or a pharmaceutically acceptable salt thereof or an ester thereof, etc. to a mammal. Another embodiment of the present invention relates to a method for enhancing or improving normal memory, the method comprising administering a compound having a stimulating activity on 1,25D₃-MARRS and/or a pharmaceutically acceptable salt thereof or an ester thereof, etc. to a mammal. The "normal memory" includes "the memory of a subject without a disease in which amyloid plaques, tau deposition, tau precipitate, PHF-tau, or neurofibrillary tangles, or Aβ (1-42)-induced axonal atrophy is observed". The compound having a stimulating activity on 1,25D₃-MARRS and/or a pharmaceutically acceptable salt thereof, the dosage form of the drug, the administration route, the content of the compound having a stimulating activity on 1,25D₃-MARRS and/or a pharmaceutically acceptable salt thereof as an active ingredient in the drug, the dosage, and the subject, etc. may be the same as above.

Another embodiment of the present invention relates to a method for activating 1,25D₃-MARRS, the method comprising administering one or more kinds selected from the group consisting of diosgenin, a diosgenin derivative, denosomin, dihydroxy vitamin D3, and a pharmaceutically acceptable salt thereof. The dosage form, the dosage, the subject, etc. may be the same as those in the administration of the compound having a stimulating activity on 1,25D₃-MARRS and/or a pharmaceutically acceptable salt thereof described above.

### EXAMPLES

Hereinafter, the present invention will be illustrated in more detail by Comparative Examples and Examples, but they are not intended to limit the present invention. The person skilled in the art can add various modifications to not only the Reference Examples and Examples shown below but also the Claims of the present application to work the invention to a maximum extent, and such modifications are included in the scope of the Claims. The present invention is based on a paper written by the present inventors, Tohda, C. et al., in Scientific Reports, 2, 535, 2012, and the content of the paper is incorporated in the present invention.

The materials, reagents, and animals to be used in the present invention may be commercially available products or be prepared by a method publicly known in this technical field.

Diosgenin to be used may be a commercial product (for example, made by Wako Pure Chemical Industries, Ltd., Tokyo Chemical Industry Co., Ltd., Sigma-Aldrich, etc.). Alternatively, diosgenin may be isolated from a natural product (for example, *Dioscorea* (yam)). Also, a diosgenin derivative to be used may be a commercial product or an extract obtainable from a natural product. Alternatively, a diosgenin derivative may be produced by chemical conversion of a saponin obtained from a natural product, or be produced by chemical synthesis using a publicly known method.

### Statistics

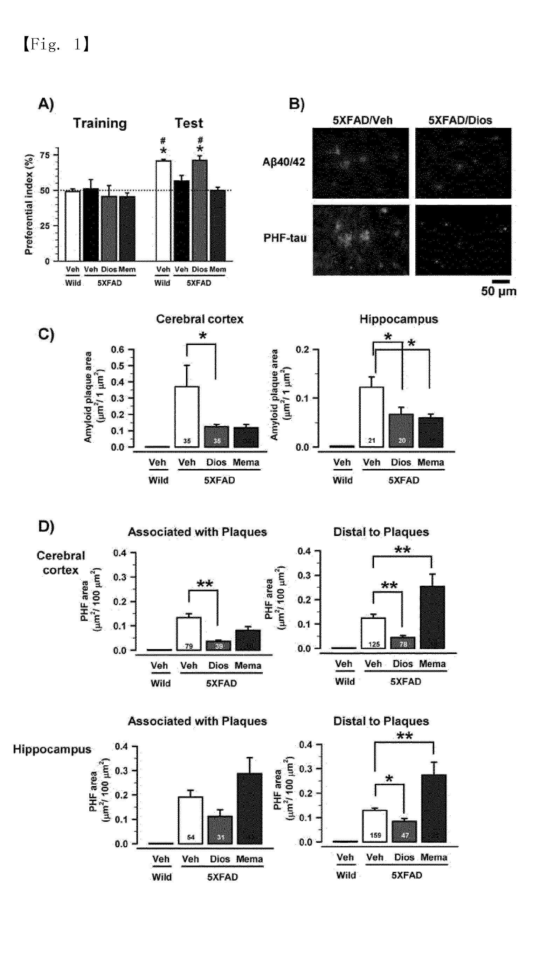

One-way analysis of variance (one-way ANOVA), post hoc Dunnett's test, and paired t-test were performed using SigmaStat 3.5 (SYSTAT, Chicago, IL, USA) and Graphpad Prism 5 (Graphpad Software, La Jolla, CA, USA). Statistical differences were considered significant when *P<0.05 or **P<0.001. The means of the data are presented together with the SE. The actual number of measured areas is shown in each column (Fig. 1C, Fig. 1D, Fig. 2B, Fig. 2C, Fig. 5A, etc.).

### Preparation of reagent etc.

Memantine hydrochloride (Tokyo Chemical Industry Co., Ltd., Tokyo, Japan) and 1α,25-dihydroxy vitamin D3 (DHVD3) (Cayman Chemicals, Ann Arbor, MI, USA) were separately dissolved in dimethyl sulfoxide (DMSO). Aβ (1-42) (Sigma-Aldrich, St. Louis, MO, USA) was dissolved in sterile distilled water and incubated at 37°C for 4 days. A phosphatidylinositol 3-kinase (PI3K) inhibitor LY294002 (Cayman Chemicals), a MEK1 inhibitor PD98059 (LC laboratories), and a protein kinase C inhibitor Goe6970 (LC laboratories, Woburn, MA, USA) were separately dissolved in DMSO. A protein kinase A inhibitor PKI (14-22) amide (myristoylated) (Enzo, Farmingdale, NY, USA) was dissolved in distilled water.

### «Example 1, Comparative Examples 1 and 2, and Reference Example 1»

The effect of diosgenin in Alzheimer's disease model mice was examined.

### Animals

Transgenic mice (5XFAD) as an animal model of AD were obtained from the Jackson Laboratory (Bar Harbor, ME, USA). The 5XFAD mice overexpress human APP695 cDNA having Swedish mutation (K670N and M671L), Florida mutation (I716V) and London mutation (V717I) and human PS1 cDNA (M146L and L286V) under the transcriptional control of the neuron-specific mouse Thy-1 promoter (Oakley, H. et al., JNeurosci, 26, 10129-10140, 2006). They were maintained by crossing hemizygous transgenic mice with B6/SJL F1 breeders.

To investigate the effect of diosgenin on 5XFAD, hemizygous 5XFAD mice (female, 6 to 8 months old) and non-transgenic littermate wild-type mice (female, 6 to 8 months old) were used. These mice were obtained by crossing hemizygous 5XFAD mice and B6/SJL F1 mice. All mice were housed with free access to food and water and were kept in a controlled environment (22 ± 2°C, 50 ± 5% humidity, 12-h light/dark cycle starting at 7:00 am).

### <Example 1>

Diosgenin was dissolved in dimethyl sulfoxide (DMSO) at 10 times the final concentration to give a stock solution. The stock solution was diluted in physiological saline to the final concentration immediately before treatment. The diosgenin solution was intraperitoneally administered to 5XFAD mice once a day (10 µmol/kg = 4.14 mg/kg) for 20 days. On the last day of administration (Day 20), each mouse was housed in a separate open-field box made of polyvinyl chloride (33 cm × 328 cm; height: 26.5 cm) and habituated to the environment for 10 min.

### <Comparative example 1>

The same procedure was performed as in Example 1 except that physiological saline containing 10% DMSO (vehicle solution) was used instead of the diosgenin solution.

### <Comparative Example 2>

Memantine hydrochloride was dissolved in DMSO at 10 times the final concentration to give a stock solution, and the stock solution was diluted in physiological saline to the final concentration immediately before treatment. Then, the same procedure was performed as in Example 1 except that the memantine hydrochloride solution (200 µmol/kg/day = 43.15 mg/kg/day) was used instead of the diosgenin solution.

### <Reference Example 1>

The same procedure was performed as in Example 1 except that physiological saline containing 10% DMSO (vehicle solution) was used instead of the diosgenin solution and that non-transgenic littermate wild-type mice were used instead of the 5XFAD mice.

### Test Example 1: Novel object recognition test

For each mouse of Example 1, Comparative Examples 1 and 2, and Reference Example 1, the path during the 10-minute habituation was tracked using a digital camera system. The distance each mouse traveled during the 10 minutes was analyzed as locomotion activity with EthoVision 3.0 (Noldus, Wageningen, The Netherlands). The next day, a novel object recognition test was performed as described in Joyashiki, E. et al., Int J Neurosci, 121, 181-190, 2011 and Tohda, C. et al., Int J Neurosci, 121, 641-648, 2011, written by the inventors and others. Testing was carried out in a dimly illuminated room. Based on the results of a preliminary test using another group of mice, an appropriate time interval between a training session and a test session was set at 30 minutes. A novel object recognition test utilizes animals' habit of showing interest in a novel object. In the training session, two identical objects are shown to have the animal memorize the objects. In the test session, two kinds of objects, that is, one of the original objects and a novel object are placed in the apparatus. The increase in the amount of time that the animal spends exploring the novel object with interest (that is, the animal remembers the original objects) is used as an index. In the present invention, the ratio (%) of the exploration time for the novel object to the total exploration time was calculated as an exploration preferential index. Wild-type mice (Reference Example 1) remember the object that has been shown once and show interest in the novel object. As a result, the index will be higher than 50%. In contrast, 5XFAD mice as AD model animals do not remember the object shown before, and the index will remain 50%. However, if the memory deficits of 5XFAD mice is improved, the index will be significantly higher than 50%.

The results of Test Example 1 are shown in Fig. 1A.

In an open field locomotion test in the training session, no significant differences were detected in the traveling speed and the distance among the four groups: the vehicle-treated wild-type mice (Reference Example 1: Wild/Veh), the vehicle-treated 5XFAD mice (Comparative Example 1: 5XFAD/Veh), the diosgenin-treated 5XFAD mice (Example 1: 5XFAD/Dios), and the memantine-treated 5XFAD mice (Comparative Example 2: 5XFAD/Mema). In the test session, however, the diosgenin-treated 5XFAD mice (Example 1: 5XFAD/Dios) showed exploratory behavior at a frequency significantly higher than 50% for the novel object (Fig. 1A). That is, after the administration of diosgenin, the 5XFAD mice as AD model animals restored interest in the novel object to the degree equal to that of the wild-type mice and the preferential index (%) was improved. In contrast, the novel object preferential index of the vehicle-treated 5XFAD mice (Comparative Example 1: 5XFAD/Veh) was 50%, which was not significantly different. Also, as is the case in the vehicle-treated 5XFAD mice (Comparative Example 1: 5XFAD/Veh), the novel object preferential index of the memantine-treated 5XFAD mice (Comparative Example 2: 5XFAD/Mema) was 50%, which was not significantly different. These result show that administration of diosgenin to the 5XFAD mice improves the mice's deficits of object recognition.

### Test Example 2: Immunohistochemistry

One day after the novel object recognition test, the mice were anesthetized and transcardially perfused with cold physiological saline. The brains were carefully removed from the skull, immediately immersed in 10 to 30% sucrose-PBS and stored at -80°C. The brains were cut in 20-µm successive coronal slices every 100 µm in the parietal area (bregma 1.4-2 mm) sections using a cryostat (CM3050S, Leica, Heidelberg, Germany). The slices were fixed with 4% paraformaldehyde and stained with a polyclonal antibody against Aβ (1-40/42) (1:300) (Chemicon, Temecula, CA, USA), a monoclonal antibody against pNF-H (1:500) (Covance, Emeryville, CA, USA), a monoclonal antibody against synaptophysin (1:500) (Sigma-Aldrich), and a monoclonal antibody against PHF-tau (Thermo Scientific, Rockford, IL, USA) at 4°C for 20 hours. Alexa Fluor 488-conjugated goat anti-mouse IgG (1:300) and Alexa Fluor 568-conjugated goat anti-rabbit antibody (1:300) were used as secondary antibodies (Molecular Probes, Eugene, OR, USA). The fluorescent images for axons, presynapses, PHF-tau and Aβ (1-40/42) were captured using a fluorescent microscope (AX-80) at 324 µm × 430 µm for pNF-H, synaptophysin and PHF-tau or at 1620 µm × 2150 µm for Aβ (1-40/42)). Three successive brain slices of the frontal cortex and five successive slices of the hippocampus were captured from a mouse for quantification. Extracellular amyloid plaques were determined by the size (greater than 50 µm in width), and the area of amyloid plaques was measured using the image analyzing software ImageJ. The area of the pNF-H-positive abnormal bulb-like axons was measured using ImageJ for every amyloid plaque in the fluorescent images (324 µm × 430 µm). The areas of the synaptophysin-positive presynaptic boutons in every amyloid plaque were measured in fluorescent images (324 µm × 430 µm) using Image J. The areas of PHF-tau in every amyloid plaque and outer amyloid plaques were measured in fluorescent images (324 µm × 430 µm) using ImageJ.

Of the results of Test Example 2, the total areas of amyloid plaques and PHF-tau per unit area of the cerebral cortex and the hippocampus are shown in Fig. 1B to Fig. 1D.

The photographs in Fig. 1B are representative images of Aβ (1-40/42)-positive plaques and PHF-tau in the cerebral cortex. It is obvious that amyloid plaques were reduced in the diosgenin-treated 5XFAD mice (Example 1: 5XFAD/Dios) as compared with the vehicle-treated 5XFAD mice (Comparative Example 1: 5XFAD/Veh). Fig. 1C shows the quantified total areas of amyloid plaques per 1 µm² in the cerebral cortex and in the hippocampus. The areas in both the cerebral cortex and in the hippocampus were significantly reduced in the diosgenin-treated 5XFAD mice (Example 1: 5XFAD/Dios) as compared with the vehicle-treated 5XFAD mice (Comparative Example 1: 5XFAD/Veh). In contrast, in the memantine-treated 5XFAD mice (Comparative Example 2: 5XFAD/Mema), the area was significantly reduced in the hippocampus but not in the cerebral cortex. Fig. 1D shows the quantified total areas of PHF-tau per 100 µm² in the cerebral cortex and in the hippocampus. The areas in both the cerebral cortex and in the hippocampus were significantly reduced in the diosgenin-treated 5XFAD mice (Example 1: 5XFAD/Dios) as compared with the vehicle-treated 5XFAD mice (Comparative Example 1: 5XFAD/Veh). In contrast, in the memantine-treated 5XFAD mice (Comparative Example 2: 5XFAD/Mema), the area in the cerebral cortex was not significantly different as compared with the vehicle-treated 5XFAD mice (Comparative Example 1: 5XFAD/Veh) and the area in the hippocampus was inversely significantly increased.

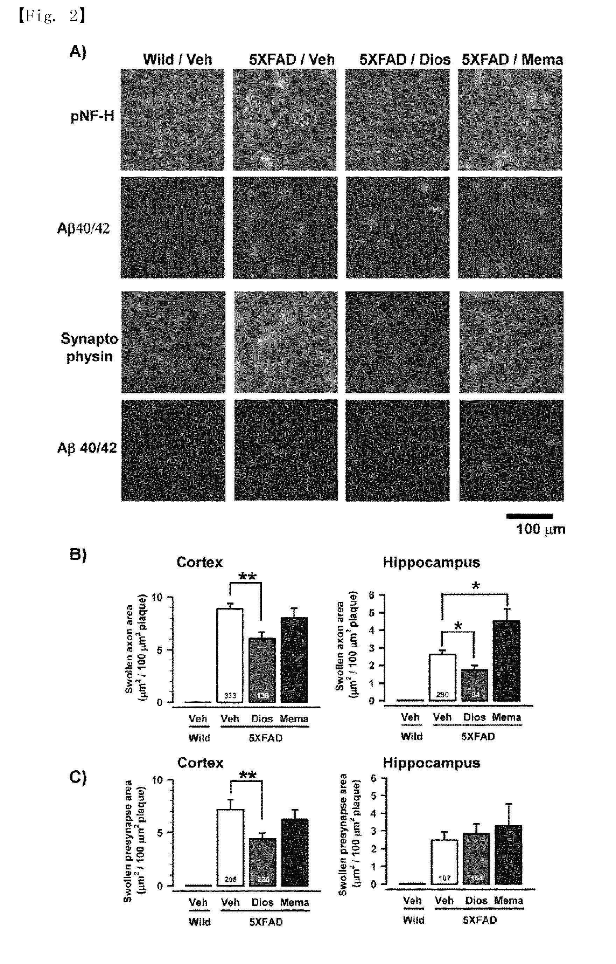

Of the results of Test Example 2, the axonal and presynaptic abnormalities associated with amyloid plaques are shown in Fig. 2A to Fig. 2C.

The photographs in Fig. 2A are representative images of the cerebral cortex of the vehicle-treated wild-type mice (Reference Example 1: Wild/Veh), the vehicle-treated 5XFAD mice (Comparative Example 1: 5XFAD/Veh), the diosgenin-treated 5XFAD mice (Example 1: 5XFAD/Dios), and the memantine-treated 5XFAD mice (Comparative Example 2: 5XFAD/Mema) as a result of double labeling with antibodies to Aβ (1-40/42) and pNF-H, or Aβ (1-40/42) and synaptophysin. The images show that amyloid plaques in the diosgenin-treated 5XFAD mice (Example 1: 5XFAD/Dios) were significantly reduced as compared with the vehicle-treated 5XFAD mice (Comparative Example 1: 5XFAD/Veh) and the memantine-treated 5XFAD mice (Comparative Example 2: 5XFAD/Mema).

Fig. 2B shows the quantified areas of abnormal axons per 100 µm² of amyloid plaque in the cerebral cortex and in the hippocampus. The vertical axis indicates swollen axon area. Abnormal axon structure, such as axonal spheroid, is found in patients with AD in its early to terminal stage (Dickson, T.C. et al., Neuroscience, 105, 99-107, 2001). The areas in both the cerebral cortex and in the hippocampus were significantly reduced in the diosgenin-treated 5XFAD mice (Example 1: 5XFAD/Dios) as compared with the vehicle-treated 5XFAD mice (Comparative Example 1: 5XFAD/Veh). In contrast, in the memantine-treated 5XFAD mice (Comparative Example 2: 5XFAD/Mema), the area in the cerebral cortex was not significantly reduced and the area in the hippocampus was inversely significantly increased.

Fig. 2C shows the quantified areas of abnormal presynapses per 100 µm² of amyloid plaque in the cerebral cortex and in the hippocampus (the vertical axis indicates swollen presynapse area). The area in the cerebral cortex significantly reduced in the diosgenin-treated 5XFAD mice (Example 1: 5XFAD/Dios) were as compared with the vehicle-treated 5XFAD mice (Comparative Example 1: 5XFAD/Veh). These results clearly show that diosgenin administration is effective in reducing the areas of abnormal axons and abnormal presynapses in the cerebral cortex and in the hippocampus.

### «Examples 2 to 7 and Comparative Examples 3 to 10»

The neurite outgrowth effect of diosgenin was examined.

### siRNA transfection

siRNAs were transfected into rat cortical neurons (SD E18) according to the manufacturer's protocol for nucleofection (Lonza, Bazel, Switzerland). In brief, rat cortical neurons (5.25 × 10⁶ cells) were mixed with 400 nM siPdia3 (a mixture of three sequences of Stealth siRNA for Pdia3, Invitrogen) or 400 nM control siRNA (Stealth RNAi Negative Control Low GC Duplex #3, Invitrogen) and electroporated with Amaxa Nucleofector (Lonza). Two days after the transfection, the cells were fixed and double-immunostained with a monoclonal antibody against a neuron marker MAP2a/2b (1:500, NeoMarker, Fremont, CA, USA) and Ab099 clone, a specific antibody for 1,25D₃-MARRS (1:500, provided by Dr. Nemere). Rat polyclonal anti-nVDR antibody (1:200, Millipore, Billerica, MA, USA) was used for the detection of the nuclear receptor for vitamin D3 (nVDR). The appropriate concentration of siRNA and the appropriate duration for knockdown (two days after transfection) were determined according to the prior art.

### <Example 2>

Untransfected cells (rat cortical neurons (SD E18)) were treated with diosgenin (1 µM) for four days.

### <Example 3>

The same procedure was performed as in Example 2 except that the cells were treated with DHVD3 (1 µM) instead of diosgenin.

### <Comparative Example 3>

The same procedure was performed as in Example 2 except that the cells were treated with a vehicle solution (0.1% DMSO) instead of diosgenin.

### <Comparative Example 4>

The same procedure was performed as in Comparative Example 3 except that cells (rat cortical neurons (SD E18)) transfected with siRNA were used instead of the untransfected cells.

### <Comparative Example 5>

The same procedure was performed as in Example 2 except that cells (rat cortical neurons (SD E18)) transfected with siRNA were used instead of the untransfected cells.

### <Comparative Example 6>

The same procedure was performed as in Example 3 except that cells (rat cortical neurons (SD E18)) transfected with siRNA were used instead of the untransfected cells.

### Test Example 3 : Measurement of axon density

The cells obtained in Examples 2 and 3, and Comparative Examples 3 to 6 were fixed with 4% paraformaldehyde and immunostained with a monoclonal antibody against an axonal marker pNF-H (1:500) and a polyclonal antibody against a neuronal marker MAP2 (1:500) at 4°C for 20 hours. Alexa Fluor 488-conjugated goat anti-mouse IgG (1:300) and Alexa Fluor 568-conjugated goat anti-rabbit IgG (1:300) were used as secondary antibodies. The fluorescent images were captured using a fluorescence microscope system (BX61/DP70, Olympus) at 324 µm × 430 µm. Twelve to fourteen images were captured. The lengths of the pNF-H-positive axons were measured using an image analyzer Neurocyte (Kurabo), which automatically traces and measures neurite length without measuring the cell bodies. The sum of the axon lengths was divided by the number of MAP2-positive neurons.

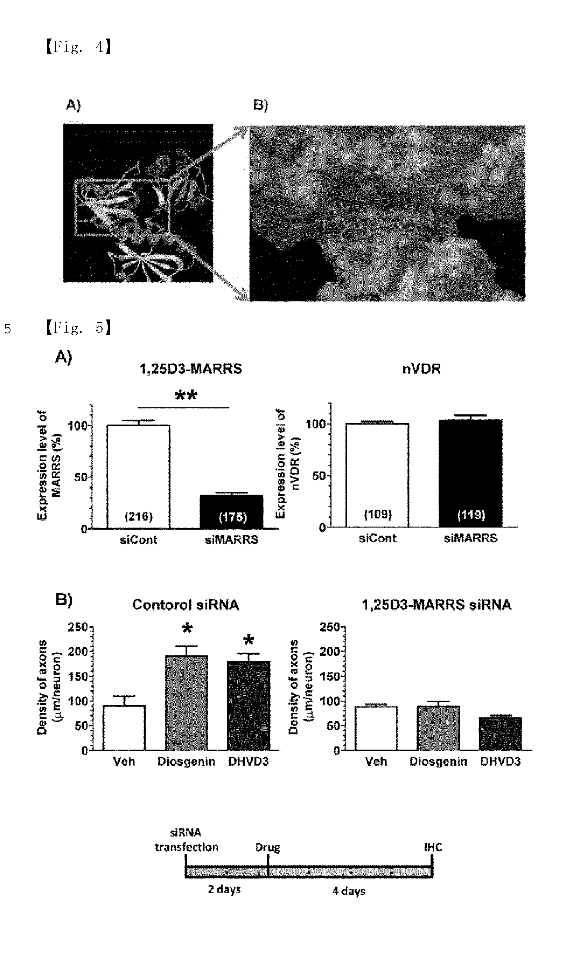

The results are shown in Fig. 5.

Fig. 5 shows the effect of 1,25D₃-MARRS knockdown on diosgenin-induced axonal outgrowth. Fig. 5A shows the quantified expression levels of 1, 25D₃-MARRS and nVDR in neurons which were transfected with siRNA for 1,25D₃-MARRS (400 nM) or control siRNA (400 nM). It was found that 1,25D₃-MARRS knockdown significantly reduced the expression level of 1,25D₃-MARRS.

Fig. 5B shows the quantified density of pNF-H-positive axons per MAP2-positive neuron for each group treated with diosgenin (1 µM: Example 2 and Comparative Example 5), DHVD3 (1 µM: Example 3 and Comparative Example 6), or a vehicle solution (Veh, 0.1%DMSO: Comparative Example 3 and Comparative Example 4). In the control groups, treatment with diosgenin (Example 2) or DHVD3 (Example 3) significantly increased the axonal density as compared with the group treated with the vehicle solution (Comparative Example 3). In the 1,25D₃-MARRS knockdown groups (Comparative Examples 4 to 6), however, diosgenin treatment and DHVD3 treatment did not show any difference as compared with the vehicle solution-treated group. Therefore, it was found that diosgenin is involved in the same receptor stimulation as that of DHVD3.

### <Example 4>

Rat cortical neurons (SD E18) were cultured for one day and treated with 1 µM diosgenin. Simultaneously, the PI3K inhibitor LY294002 (10 µM), the MEK1 inhibitor PD98059 (1 µM and 10 µM), the protein kinase C inhibitor Goe6970 (10 nM and 100 nM), or the protein kinase inhibitor PKI (14-22) amide (myristoylated) (0.1 µM and 1 µM) was added to the cells.

### <Example 5>

The same procedure was performed as in Example 4 except that DHVD3 (1 µM) was used instead of diosgenin (1 µM).

### Test Example 4: Measurement of the effects of protein kinase inhibitors on diosgenin-induced axonal growth

Four days after the treatment in Examples 4 and 5, the cells were fixed and double-immunostained for pNF-H and MAP2. The density of pNF-H-positive axons per MAP2-positive neuron was quantified for each treatment. The measurement of axon density was performed as in the above Test Example 3.

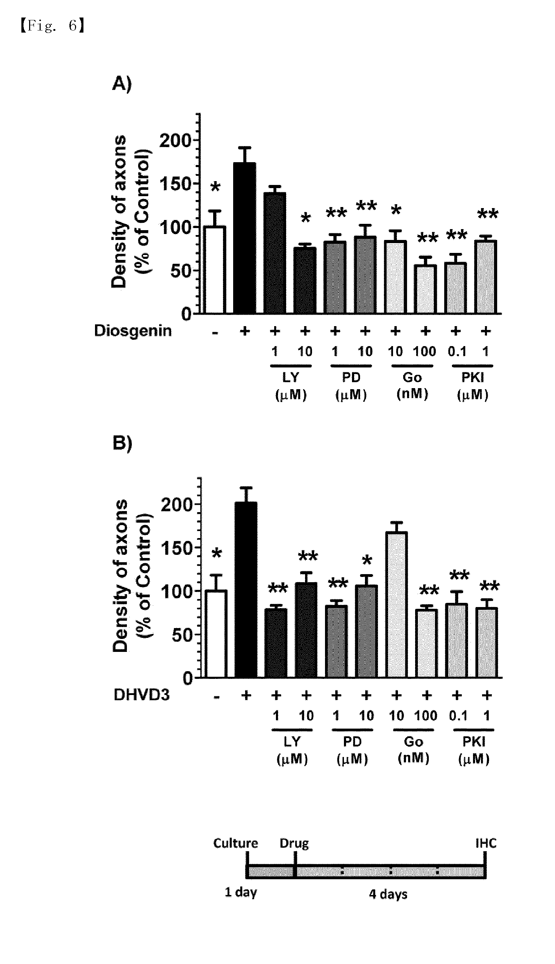

The results are shown in Fig. 6.

Fig. 6A shows that all the protein kinase inhibitors used significantly inhibited diosgenin-induced axonal growth. Also, Fig. 6B shows that all the protein kinase inhibitors used significantly inhibited DHVD3-induced axonal growth. This fact supports that diosgenin is involved in the same receptor stimulation as that of DHVD3.

### <Example 6>

Aβ (1-42) was incubated at 37°C for four days for aggregation beforehand. Then, cells (rat cortical neurons (SD E18)) were treated with Aβ (1-42) for 3 days. After the 3-day treatment, a control antibody (normal rabbit IgG, 1:5000) was added to the cells, and the cells were incubated for 10 minutes and further treated with diosgenin (0.1 µM) for 5 days.

### <Example 7>

The same procedure was performed as in Example 6 except that the cells were treated with diosgenin (1 µM).

### <Comparative Example 7>

The same procedure was performed as in Example 6 except that the cells were treated with a vehicle solution (0.1% DMSO) instead of diosgenin.

### <Comparative Example 8>

The same procedure was performed as in Comparative Example 7 except that polyclonal anti-rabbit 1,25D₃-MARRS (Ab099 clone, 1:500) was used instead of the control antibody.

### <Comparative Example 9>

The same procedure was performed as in Example 6 except that polyclonal anti-rabbit 1,25D₃-MARRS (Ab099 clone, 1:500) was used instead of the control antibody.

### <Comparative Example 10>

The same procedure was performed as in Example 7 except that polyclonal anti-rabbit 1,25D₃-MARRS (Ab099 clone, 1:500) was used instead of the control antibody.

### <Reference Example 2>

Rat cortical neurons (SD E18) not treated with any antibody or drug were prepared for the test.

### Test Example 5: Measurement of axon density

The cells obtained in Examples 6 and 7, and Comparative Examples 7 to 10, and the cells of Reference Example 2 were fixed with 4% paraformaldehyde and immunostained with a monoclonal antibody against an axonal marker pNF-H (1:500) and a polyclonal antibody against a neuronal marker MAP2 (1:500) at 4°C for 20 hours. Alexa Fluor 488-conjugated goat anti-mouse IgG (1:300) and Alexa Fluor 568-conjugated goat anti-rabbit IgG (1:300) were used as secondary antibodies. The fluorescent images were captured using a fluorescence microscope system (BX61/DP70, Olympus) at 324 µm × 430 µm. Seven to nine images were captured. The lengths of the pNF-H-positive axons were measured using an image analyzer Neurocyte (Kurabo), which automatically traces and measures neurite length without measuring the cell bodies. The sum of the axon lengths was divided by the number of MAP2-positive neurons.

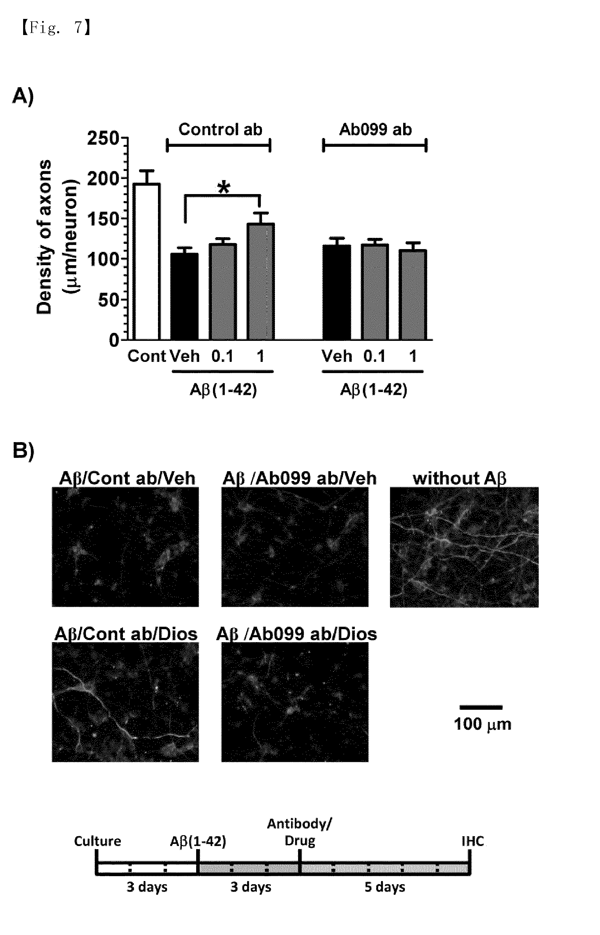

The results are shown in Fig. 7.

Figs. 7A and 7B show that in the cells where the function of 1,25D₃-MARRS was inhibited by the neutralizing antibody, diosgenin-induced axonal growth was significantly suppressed. This fact shows that 1,25D₃-MARRS is involved in the diosgenin-induced axonal growth.

### Reference Test 1: Identification of the target protein for diosgenin

### Primary culture

Embryos were removed from pregnant Sprague-Dawley rats (Japan SLC, Shizuoka, Japan) at 17 days of gestation. The cortices were dissected, and the dura mater was removed. The tissues were minced, dissociated and grown in cultures with Neurobasal medium (Invitrogen, Grand Island, NY, USA) containing 12% B-27 supplement (Invitrogen), 0.6% D-glucose, and 2 mM L-glutamine on 8-well chamber slides (Falcon, Franklin Lakes, NJ, USA) coated with 5 mg/mL poly-D-lysine at 37°C in a humidified incubator with 10% CO₂. The seeding cell density was 4.35 × 10⁴ cells/cm²

### Detection of luminescence resulting from 1,25D₃-MARRS binding

Primary cultured cortical neurons (SD, E17) were harvested into a 96-well white plate at a density of 0.5 × 10⁵ cells/well. Three days later, the cells were treated with DHVD3 alone or with a combination of DHVD3 and diosgenin at 37°C for 10 minutes. The cells were fixed with 4% paraformaldehyde and incubated at 4°C for 20 hours with a monoclonal antibody against DHVD3 (1:2000, Acris Antibodies, Herford, Germany) in Triton-X-free PBS to detect only cell surface-binding DHVD3. Horse radish peroxidase-conjugated goat anti-mouse IgG (1:2000) was used as a secondary antibody. The chemiluminescence of the antigen-antibody complex was detected using a GENios multi-plate reader (Tecan, Mannedorf, Zurich, Switzerland).

A mouse cortical neuron primary culture (ddY, E14) was maintained for 24 hours without any drug treatment. The cells were lysed with M-PER (Thermo Scientific) supplemented with protease and phosphatase inhibitors. After centrifuged (20,000 × g) for 15 minutes, the protein concentration of the lysates was determined using Quick Start (Bio-Rad Laboratories, Hercules, CA, USA). The cell lysate (5.6 µg each) was added to 2 µL of a vehicle solution of 10 mM diosgenin and incubated at 4°C for 30 minutes. The mixture was proteolyzed using 7.5 µg of thermolysin in a reaction buffer [50 mM Tris·HCl (pH 8.0), 50 mM NaCl, and 10 mM CaCl₂] at room temperature for 10 minutes. To stop the proteolysis, 0.5 M EDTA (pH 8.0) was added to each sample at a ratio of 1:10. The samples were then mixed well and placed on ice. The reaction samples were electrophoresed using a 5 to 20% real gel plate (Bio Craft, Tokyo, Japan). The separated proteins were stained using a negative gel stain MS kit (Wako). The bands in the diosgenin-treated lysate that were thicker than those in the vehicle-treated lysate were cut out and prepared for mass spectrometry analysis. After alkylation using iodoacetamide and in-gel digestion by trypsin, the supernatants were analyzed by nano-LC/MS/MS using LTQ Orbitrap XL (ThermoFisher). Based on the obtained MS/MS data, the corresponding protein was searched for using Mascot (www.matrixscience.com).

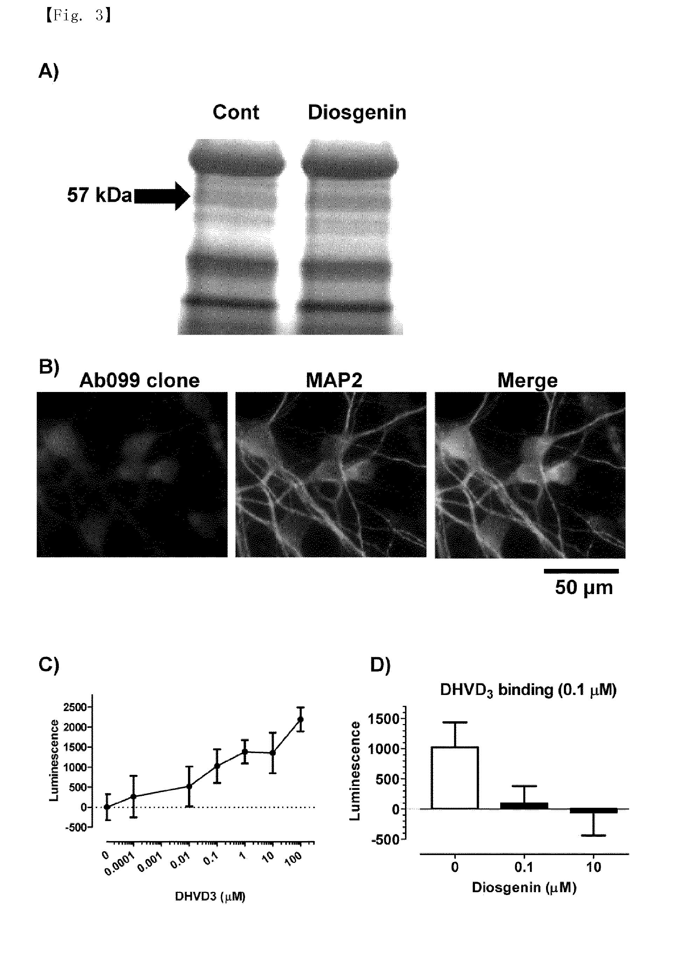

Fig. 3 reveals that the direct target protein of diosgenin was 1, 25D₃-MARRS, which will be described in more detail below. Fig. 3A shows a silver-stained SDS-PAGE gel. The band in the diosgenin-treated lysate (diosgenin lane) thicker than that in the vehicle-treated lysate (Cont lane) was cut out and prepared for mass spectrometry analysis. LC/MS/MS analysis indicated that the band was the protein disulfide isomerase A3 (Pdia3), that is, 1,25D₃-MARRS. Fig. 3B shows the images as a result of double labeling with antibodies against 1,25D₃-MARRS (Ab099 clone) and MAP2 in the primary culture of the rat cortical neurons. Fig. 3C shows the dose-dependent increase in chemiluminescence intensity, resulting from the dose-dependency of cell surface binding of DHVD3 in the primary culture cortical neurons. Fig. 3D shows replacement of DHVD3 with diosgenin in the binding to the cell surface. That is, diosgenin (0.1 µM or 10 µM) was added to the cells with DHVD3 (0.1 µM) and incubation was performed for 10 minutes. As a result, the immunochemiluminescence intensity was remarkably decreased. This means replacement of DHVD3 with diosgenin in the binding to the cell surface.

### Reference Test 2: Docking simulation

Autodock 4.0 (AutoDock4 and AutoDockTools4; Molecular Graphics Laboratory, Department of Molecular Biology The Scripps Research Institute) was used to predict the conformation of the protein-ligand complexes. Ligand structures (diosgenin and DHVD3) were constructed and minimized using MOPAC2009 for 2D and 3D conformation. For the docking studies, the crystal structures of the proteins (PDB ID: NP_036630.1 for RLAR; NP_001619.1 for human AR) were determined from the protein sequence alignment [Brookhaven Protein Data Bank (PDB ID: 3f8u chain A for 1,25D₃-MARRS; 1ie9 chain A for nVDR)]. The predicted protein-ligand complexes were optimized and ranked according to the empirical scoring function, which estimates the binding free energy of the ligand-receptor complex.

Fig. 4 shows the docking simulation of diosgenin to 1,25D₃-MARRS. Fig. 4A shows the predicted binding site in the complex of 1,25D₃-MARRS and diosgenin. Fig. 4B shows that the groove area between the a domain and the b domain of 1,25D₃-MARRS is in contact with diosgenin.

As shown in Table 1, the docking scores of diosgenin and DHVD3 to 1,25D₃-MARRS were -8.4 and -7.9, respectively, and the docking scores of diosgenin and DHVD3 to nVDR were -8.2 and -12.6, respectively. These results suggest that the binding potency of diosgenin to 1,25D₃-MARRS is slightly higher than that of DHVD3 and that the binding potency of DHVD3 to nVDR is much higher than that of diosgenin.

**Table 1**

| Docking scores of ligands and receptors | | |
|---|---|---|
| | Diosgenin | DHVD3 |
| 1,25D₃-MARRS | -8.4 | -7.9 |
| nVDR | -8.2 | -12.6 |

As shown above, diosgenin administration to 5XFAD mice markedly improved the object recognition memory. Diosgenin administration markedly decreased amyloid plaques and neurofibrillary tangles in the cortex and hippocampus. Diosgenin administration markedly decreased neuronal degeneration and presynaptic degeneration observed only in regions closely associated with amyloid plaques. In the above tests, 1,25D₃-MARRS was identified as a target protein for diosgenin. Knockdown of 1,25D₃-MARRS completely inhibited diosgenin-induced axonal growth in cortical neurons. Treatment with a neutralizing antibody against 1,25D₃-MARRS diminished the axonal regeneration effect of diosgenin in Aβ(1-42)-induced axonal atrophy. This demonstrated for the first time that diosgenin stimulates 1, 25D₃-MARRS and activates signaling pathway. Herein, diosgenin was used in the Examples, but other compounds (DHVD3, denosomin) having a stimulating activity on 1,25D₃-MARRS are also effective against AD. Therefore, this novel mechanism and pathway provide a completely new technical idea leading to anti-AD therapy. It was also demonstrated for the first time by a biological test that the above-described compounds, such as diosgenin, have a stimulating activity on 1,25D₃-MARRS and have an excellent preventive and/or therapeutic effect on Alzheimer's disease.

### «Examples 8 to 12 and Comparative Examples 11 to 15»

The memory enhancing effect of diosgenin in normal mice was examined.

### Animals

ddY mice and C57BL/6 mice were obtained from Japan SLC, Inc., Hamamatsu, Japan. All mice were housed with free access to food and water and were kept in a controlled environment (22 ± 2°C, 50 ± 5% humidity, 12-h light/dark cycle starting at 7:00 am).

### <Example 8>

Diosgenin (Wako Pure Chemical Industries, Ltd., Osaka, Japan) was dissolved in ethanol at 10 times the final concentration to give a stock solution, and the stock solution was diluted in a 5% aqueous solution of glucose to the final concentration. The diosgenin solution was intraperitoneally administered to normal mice (ddY mice, male, 6 weeks old) once a day (10 µmol/kg/day) for 5 or 6 days. One hour after the last administration, each mouse was housed in a separate open-field box made of polyvinyl chloride (33 cm × 28 cm; height: 26.5 cm), habituated to the environment for 10 minutes, and used in the test.

### <Comparative Example 11>

The same procedure was performed as in Example 8 except that a 10% ethanol solution (vehicle solution) containing 5% glucose was used instead of the diosgenin solution.

### Test Example 6: Novel object recognition test

Using the mice of Example 8 and Comparative Example 11, a novel object recognition test was performed to evaluate the object recognition memory. Testing was carried out in a dimly illuminated room (100 lux). The test was performed as described in Joyashiki, E. et sl., Int J Neurosci, 121, 181-190, 2011 and Tohda, C. et al., Int J Neurosci, 121, 641-648, 2011, written by the inventors and others, except that the time interval between a training session and a test session was 48 hours. As a locomotion index, the path of each mouse was tracked using a digital camera system. The distance each mouse traveled during the 10 minutes was analyzed as locomotion activity with EthoVision 3.0 (Noldus, Wageningen, The Netherlands).

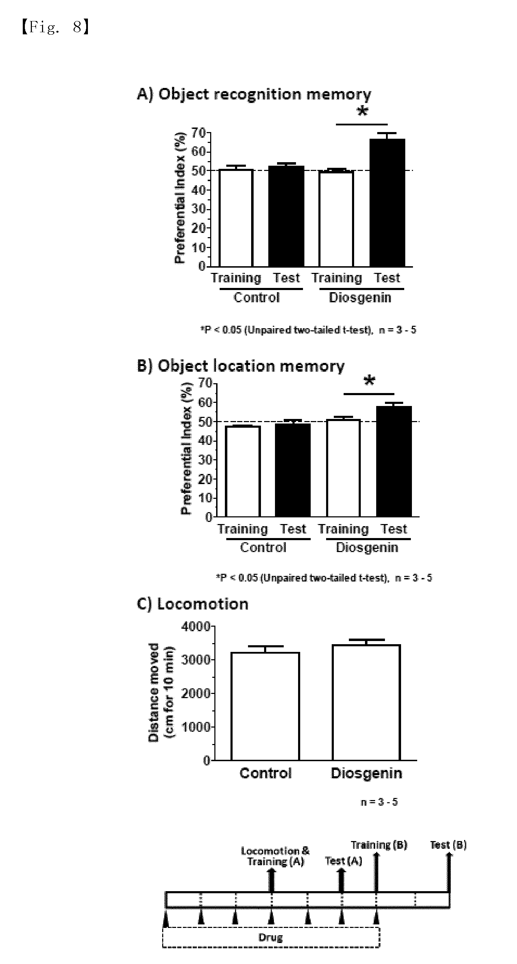

The results are shown in Fig. 8A.

The diosgenin-treated mice (Example 8) showed frequent exploratory behavior for the novel object, and the behavior index was significantly higher than 50%, the chance level. The frequency of the exploratory behavior for the novel object in the control mice (Comparative Example 11) was close to 50%.

### Test Example 7: Location recognition test

Intraperitoneal administration to the mice of Example 2 and Comparative Example 3 was performed for 7 days. One hour after the last administration, a location recognition test was performed to evaluate the spatial recognition memory. Testing was carried out in a dimly illuminated room (100 lux). The test was performed as described in Tohda, C. et al., Br J Pharmacol. 157, 1427-1440, 2009, written by the inventor and others, except that the time interval between a training session and a test session was 48 hours. As a locomotion index, the path of each mouse was tracked using a digital camera system. The distance each mouse traveled during the 10 minutes was analyzed as locomotion activity with EthoVision 3.0 (Noldus, Wageningen, The Netherlands).

The results are shown in Fig. 8B.

In cases where an object with which the mouse had become familialized during the training session was moved to a new location in the test session, the diosgenin-treated mice (Example 8) showed exploratory behavior at a frequency significantly higher than the chance level (50%). In contrast, the control mice (Comparative Example 11) did not show unique exploratory behavior for the object moved to a new location.

### Test Example 8: Measurement of spontaneous motor activity

The spontaneous motor activity was evaluated based on the distance each mouse traveled in an open field during 10 minutes.

The results are shown in Fig. 8C. There was no significant difference between Example 8 and Comparative Example 11. The results show that there is no sign of adverse effect caused by diosgenin administration.

### <Example 9>

The diosgenin solution was intraperitoneally administered to normal mice (C57BL/6, male, 8 weeks old) (100 µmol/kg/day) for 14 days. On the day after the 14th administration, the mice were used in the test.

### <Comparative Example 12>

The same procedure was performed as in Example 9 except that a 10% ethanol solution (vehicle solution) containing 5% glucose was used instead of the diosgenin solution.

### Test Example 9: Novel object recognition test

Using the mice of Example 9 and Comparative Example 12, the novel object recognition test was performed. The test was performed under the conditions as described above.

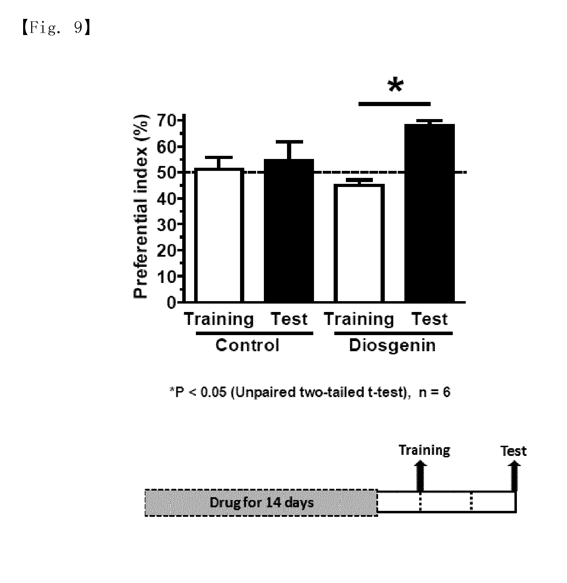

The results are shown in Fig. 9. Diosgenin administration significantly improved the object recognition memory.

### <Example 10>

Dioscin in an amount of 30 µmol/kg/day was orally administered to normal mice (ddY mice, male, 6 weeks old) for 4 days. Dioscin (Chromadex, Irvine, CA, USA) was dissolved in ethanol at 10 times the final concentration to give a stock solution, and the stock solution was diluted in a 5% aqueous solution of glucose to the final concentration. One hour after the last administration, the mice were used in the test.

### <Comparative Example 13>

The same procedure was performed as in Example 10 except that dioscin was not administered.

### Test Example 10: Novel object recognition test

Using the mice of Example 10 and Comparative Example 13, the novel object recognition test was performed. The test was performed under the same conditions as in the above Test Example 6.

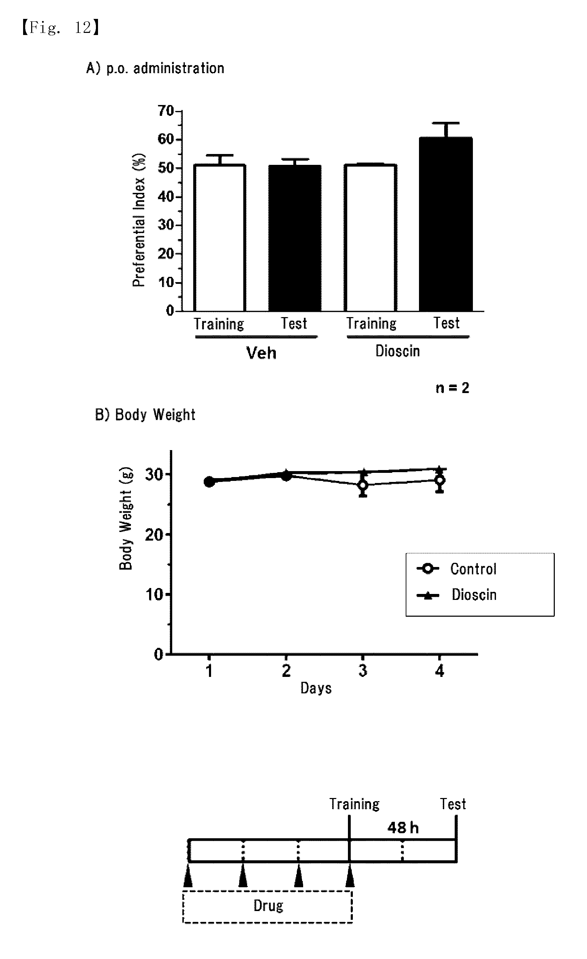

The results are shown in Fig. 12A. The dioscin (30 µmol/kg/day)-treated mice (Example 10) showed frequent exploratory behavior for the novel object, and the behavior index was significantly higher than 50%, the value of behavior selected by chance (Fig. 12A). The frequency of the exploratory behavior for the novel object in the control mice (Comparative Example 13) was similar to the chance level (50%).

There was no significant difference in the transition of body weight among the administration groups (Fig. 12B).

### Test Example 11: Continuous administration of 1,25D₃-MARRS neutralizing antibody

### <Comparative Example 14>

To neutralize the 1,25D₃-MARRS function of normal mice (ddY mice, male, 6 weeks old), a polyclonal antibody specific to 1,25D₃-MARRS, i.e., Ab099 clone, was intraventricularly administered in a continuous manner. Ab099 diluted in ACSF (artificial cerebrospinal fluid) was charged into a micro osmotic pump (model: 1007D, Alzet, Cupertino, CA, USA) and a brain infusion kit 3 (Alzet). The dilution rate of Ab099 was determined depending on the cerebrospinal fluid (CSF) volume and the inflow velocity so that the concentration would be sufficient for Ab099 to act as a neutralizing antibody in CSF. Using stereotaxic apparatus, a brain infusion cannula (brain infusion kit) was inserted into the right ventricle of each mouse anesthetized beforehand (anterior/posterior: -0.22 mm posterior from bregma, medial/lateral: +1.0 mm, dorsal/ventral: -2.5 mm below surface). The entire cannula insertion site was covered with Loctite 454 (Alzet). The pump was subcutaneously implanted in the back of the mouse. The incision in the skin was sutured.

After the start of continuous antibody administration, the intraperitoneal administration of diosgenin was initiated. After 5-day administration of diosgenin, the object recognition memory training was initiated. The next day, the last administration of diosgenin was performed. The test session was initiated 48 hours after the start of the training session.

### <Comparative Example 15>

The same procedure was performed as in Comparative Example 14 except that ACSF (artificial cerebrospinal fluid) was intraventricularly administered instead of the Ab099 clone and that a vehicle solution was intraperitoneally administered instead of diosgenin.

### <Example 11>

The same procedure was performed as in Comparative Example 14 except that ACSF (artificial cerebrospinal fluid) was intraventricularly administered instead of the Ab099 clone.

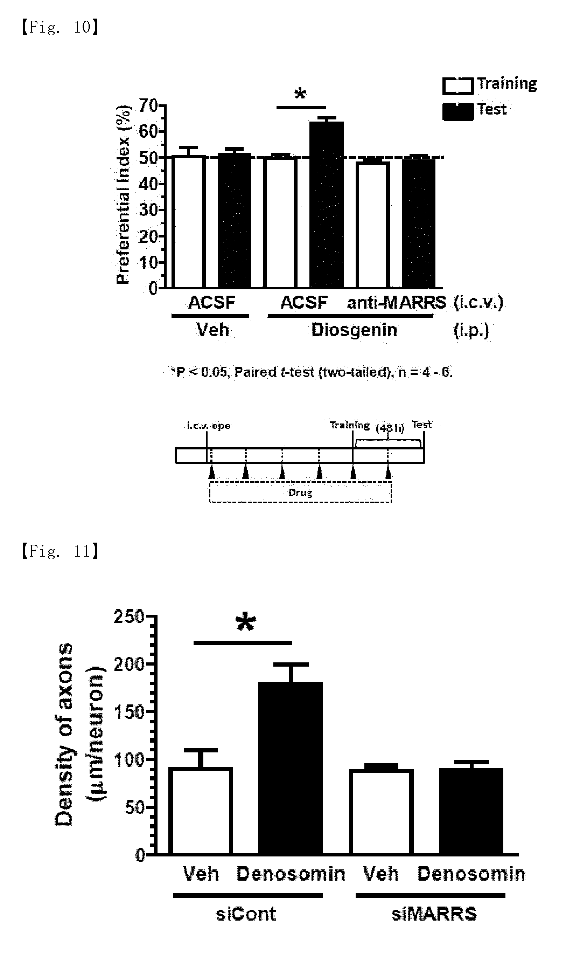

The results are shown in Fig. 10.

In the test session, the mice of Comparative Example 15 did not show improvement of the object recognition memory. In contrast, the preferential index of the mice treated with ACSF and diosgenin (Example 11) was significantly improved, showing that the memory was improved. In the mice to which the 1,25D₃-MARRS neutralizing antibody had been injected (Comparative Example 14), the memory improvement attributable to diosgenin was completely offset. These data show diosgenin activates 1,25D₃-MARRS and thereby improves normal memory.

### Reference Test Example 3: Confirming that denosomin is also an exogenous stimulator of 1,25D₃-MARRS

Denosomin also has an axonal elongation activity similar to that of diosgenin. The inventors investigated whether the effect of Denosomin is also mediated by 1,25D₃-MARRS. 1,25D₃-MARRS was knocked down using siRNA transfection. Two days after the transfection of siRNA for 1, 25D₃-MARRS (400 nM), the 1,25D₃-MARRS level was markedly reduced in cortical neurons (Fig. 5A). In contrast, nVDR expression in cortical neurons was not changed by the transfection of siRNA for 1,25D₃-MARRS (400 nM), suggesting that the knockdown did not affect the genomic signaling of DHVD3 (Fig. 5A). Two days after the siRNA transfection, denosomin (1 µM) or a vehicle solution was applied to the cells. Further, after 4-day incubation, the cells were double-immunostained for pNF-H and MAP2 to measure the axonal length per neuron. In cortical neurons, after the transfection of control siRNA, the density of axons was significantly increased by denosomin administration (Fig. 11). However, denosomin-induced axonal growth was almost completely inhibited in neurons after the transfection of 1,25D₃-MARRS siRNA (Fig. 11). The data indicate that 1,25D₃-MARRS is essential for the denosomin-induced axonal growth.

### «Example 12, Comparative Example 16, and Reference Example 3»

The effect of dioscin (diosgenin glycoside) contributing to the improvement of the object recognition memory in an Alzheimer's disease model (5XFAD mice) was examined.

### <Example 12>

Dioscin (30 µmol/kg/day) was orally administered to 5XFAD mice (male, 20 weeks old) for 6 days. One hour after the last administration, the training session was initiated.

### <Comparative Example 16>

The same procedure was performed as in Example 12 except that a vehicle solution was administered instead of dioscin.

### <Reference Example 3>

The same procedure was performed as in Comparative Example 16 except that littermate wild-type mice were used instead of the 5XFAD mice.

### Test Example 12: Novel object recognition test

Using the mice of Example 12 and Comparative Example 16, a novel object recognition test was performed to evaluate the object recognition memory. The test was performed under the same conditions as in Test Example 6 except that the time interval between a training session and a test session was one hour.

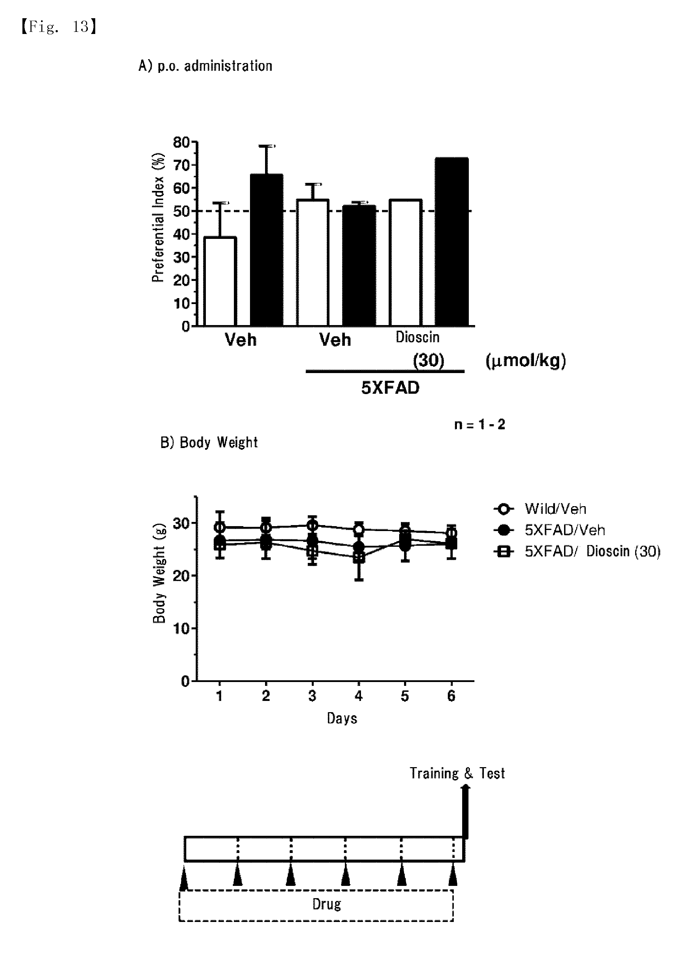

The results are shown in Fig. 13.

In the novel object recognition test, the vehicle solution-treated wild-type mice (Reference Example 3) and the dioscin (30 µmol/kg/day)-treated 5XFAD mice (Example 12) showed frequent exploratory behavior for the novel object, and the behavior index was markedly higher than 50%, the value of behavior selected by chance (Fig. 13A). The frequency of the exploratory behavior for the novel object in the vehicle solution-treated 5XFAD mice (Comparative Example 16) was similar to the chance level (50%).

None of the groups showed marked change in the body weight (Fig. 13B).

### «Example 13 and Comparative Example 17»

To confirm the effect of diosgenin on the hindlimb function of spinal cord injury mice, a spinal cord injury model test was performed.

### Spinal cord injury (SCI) mice

In the spinal cord injury model test, 7-week-old female ddY mice (SLC) were used. The mice were maintained with free access to food and water in a controlled environment (22±2°C, 50 ± 5% humidity, 12-h light/dark cycle starting at 7:00 am). A contusion was created by dropping a 3.0 g weight once onto an exposed first lumbar vertebrae (L1) of each mouse from a height of 3 cm using stereotaxic apparatus (made by Narishige). One hour after surgery, the mice were randomly divided into a vehicle solution-treated group and a diosgenin-treated group, and then drug administration was initiated.

### <Example 13>

Diosgenin (10 µmol/kg) was intraperitoneally administered to SCI mice once a day for 7 days.

### <Comparative Example 17>

The same procedure was performed as in Example 13 except that a vehicle solution was used instead of diosgenin.

### Test Example 13: Evaluation of hindlimb motor function based on TMS

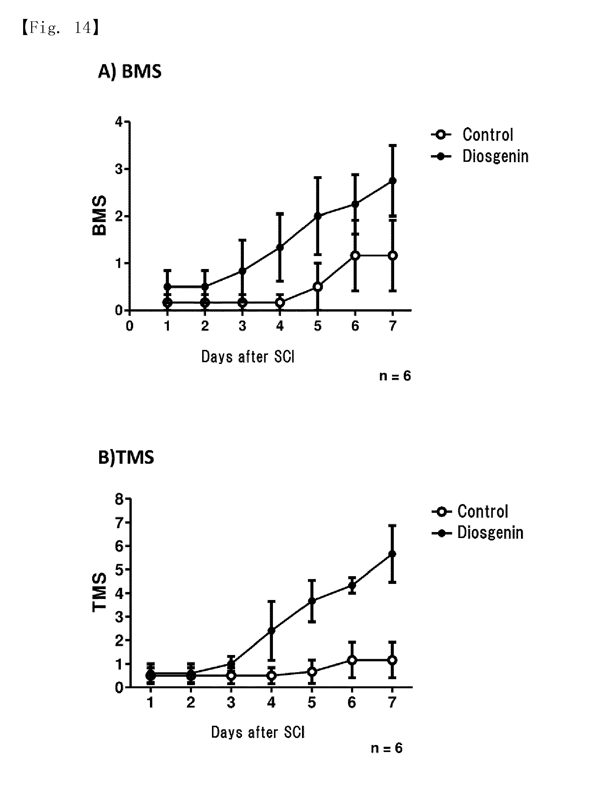

One hour after the last administration in Example 13 and Comparative Example 17, each mouse was moved to an open field (23.5 cm × 16.5 cm × 12.5 cm) and observed for behavior evaluation for 5 minutes. The locomotion behavior in the open field was evaluated using the Basso Mouse Scale (BMS), which is a general standard for evaluation of the hindlimb function of SCI model mice (Fig. 14A), and a 0-18-point Toyama Mouse Scale (TMS), which is a modified BMS devised by the inventors for better test accuracy, (Fig. 14B).

The new Toyama Mouse Scale (TMS), which is a modified BMS devised by the inventors for better test accuracy, was used in the hindlimb function of SCI mice. The TMS is shown in Table 2.

**[Table 2]**

| Item | 1 | | 2 | 3 | 4 | | 5 | 6 | 7 |
|---|---|---|---|---|---|---|---|---|---|
| | Ankle | | Joints other than ankle | Motion of digits | Contact of Plantar | | Weight support | Coordination | Paw position |
| | Frequency | Range of Motion | Frequency | | At rest | While stepping | BSS | | Sweeping motion |
| | No movement (0) | No movement (0) | No movement (0) | No movement (0) | No contact (0) | No contact (0) | No weight support (0) | No coordination (0) | No sweeping (0) |
| | 50% or less (1) | 50% or less (1) | Move (1) | Move (1) | Partial contact (1) | Partial contact (1) | Occasional hip lifting (1) | 80% or more (1) | Rotate (1) |
| | 50% or more (2) | 50% or more (2) | | | Heel contacts (2) | Entire plantar including heel contacts with frequency of 50% or less (2) | Unstable stepping with hip lifted (2) | | Parallel (2) |
| | | | | | | Entire plantar including heel contacts with frequency of 50% or more (3) | Almost consistent weight-supported stepping (3) | | |
| | | | | | | Consistent contact of entire plantar including heel (4) | | | |

| | | | | | | | | | |
|---|---|---|---|---|---|---|---|---|---|
| The numbers in parentheses in the table are scores. Each item score is determined first, and the sum is figured out for evaluation. | | | | | | | | | |

In both Example 13 and Comparative Example 17, the number of tested mice was 3, and therefore, 6 hindlimbs in total were evaluated (n = 6). The results are shown in Fig. 14.

The diosgenin-treated mice (Example 13, the group indicated by the black circle in Fig. 14) showed improved hindlimb motor function as compared with the vehicle solution-treated mice (Comparative Example 17, the group indicated by the white circle in Fig. 14).

### Industrial Applicability

The present invention can provide a compound and a pharmaceutical composition for radically preventing and/or treating a neurological disease, such as Alzheimer's disease, dementia, Parkinson's disease, spinal cord injury, or brain contusion and provide a method for the treatment.

## Claims

1. A compound for prevention and/or treatment of a neurological disease, the compound having a stimulating activity on 1,25D₃-MARRS; and/or a pharmaceutically acceptable salt thereof, with the exception of the case where the disease is Alzheimer's disease or spinal cord injury and the compound is denosomin.

2. The compound according to claim 1 and/or a pharmaceutically acceptable salt thereof, wherein the compound having a stimulating activity on 1,25D₃-MARRS is diosgenin and/or a diosgenin derivative.

3. The compound according to claim 1 and/or a pharmaceutically acceptable salt thereof, wherein the compound having a stimulating activity on 1,25D₃-MARRS is diosgenin and/or dioscin.

4. The compound according to any of claims 1 to 3 and/or a pharmaceutically acceptable salt thereof, wherein the neurological disease is Alzheimer's disease, dementia, Parkinson's disease, spinal cord injury, or brain contusion.

5. Use of the compound according to any of claims 1 to 3 and/or a pharmaceutically acceptable salt thereof for the production of a drug for preventing and/or treating a neurological disease.

6. The use according to claim 5, wherein the neurological disease is Alzheimer's disease, dementia, Parkinson's disease, spinal cord injury, or brain contusion.

7. The compound according to any of claims 1 to 3 and/or a pharmaceutically acceptable salt thereof for the use in the prevention and/or treatment of a neurological disease.

8. The compound according to claim 7 and/or a pharmaceutically acceptable salt thereof, wherein the neurological disease is Alzheimer's disease, dementia, Parkinson's disease, spinal cord injury, or brain contusion.

9. A pharmaceutical composition for the treatment of a neurological disease, the composition comprising a therapeutically effective amount of the compound according to any of claims 1 to 3 and/or a pharmaceutically acceptable salt thereof.

10. The pharmaceutical composition according to claim 9, wherein the neurological disease is Alzheimer's disease, dementia, Parkinson's disease, spinal cord injury, or brain contusion.

11. The pharmaceutical composition according to claim 9 or 10, the composition further comprising a therapeutically effective amount of one or more compounds known to be effective for the treatment or prevention of a disease or one or more pharmaceutically acceptable salts thereof.

12. The pharmaceutical composition according to claim 11, wherein the one or more compounds known to be effective for the treatment or prevention of a disease or one or more pharmaceutically acceptable salts thereof are known to be effective for the treatment or prevention of a neurological disease.

13. A method for preparing the pharmaceutical composition according to any of claims 9 to 12, the method comprising using at least one carrier.

14. A method for preventing and/or treating a neurological disease, the method comprising administering the compound according to any of claims 1 to 3 or a pharmaceutically acceptable salt thereof to a mammal including a human.

15. The method according to claim 14, wherein the compound according to any of claims 1 to 3 or a pharmaceutically acceptable salt thereof is used in combination with one or more compounds known to be effective for the treatment or prevention of a neurological disease or one or more pharmaceutically acceptable salts thereof.

16. The method according to claim 14 or 15, wherein the neurological disease is Alzheimer's disease, dementia, Parkinson's disease, spinal cord injury, or brain contusion.

17. A kit used for preventing and/or treating a neurological disease, the kit comprising the compound according to any of claims 1 to 3 or a pharmaceutically acceptable salt thereof.

18. The kit according to claim 17, comprising the compound according to any of claims 1 to 3 or a pharmaceutically acceptable salt thereof and a container.

19. A method for activating 1,25D₃-MARRS, the method comprising administering one or more kinds selected from the group consisting of diosgenin, a diosgenin derivative, denosomin, dihydroxy vitamin D3, and a pharmaceutically acceptable salt thereof.

20. A method for preventing or treating Alzheimer's disease, the method comprising administering one or more kinds selected from the group consisting of diosgenin, a diosgenin derivative, dihydroxy vitamin D3, and a pharmaceutically acceptable salt thereof.

21. A method for reducing amyloid plaques, tau deposition, tau precipitate, PHF-tau, or neurofibrillary tangles, the method comprising administering diosgenin, a derivative thereof, a pharmaceutically acceptable salt thereof, or an ester thereof to a mammal including a human.

22. A method for suppressing Aβ (1-42)-induced axonal atrophy, the method comprising administering diosgenin, a derivative thereof, a pharmaceutically acceptable salt thereof, or an ester thereof to a mammal including a human.

23. A method for activating a signaling pathway by the stimulation of 1,25D₃-MARRS, the method comprising administering diosgenin, a derivative thereof, a pharmaceutically acceptable salt thereof, or an ester thereof to a mammal including a human.

24. A supplement, a health food, a functional food, or a specified health food comprising the compound according to any of claims 1 to 3 and/or a pharmaceutically acceptable salt thereof.
